(19) Europäisches Patentamt
European Patent Office
Office européen des brevets

(11) Veröffentlichungsnummer: **0 508 190 B1**

(12) **EUROPÄISCHE PATENTSCHRIFT**

(45) Veröffentlichungstag der Patentschrift: **22.11.95**

(21) Anmeldenummer: **92104937.5**

(22) Anmeldetag: **21.03.92**

(51) Int. Cl.[6]: **C07D 239/06**, C07D 233/22, C07D 233/20, C07D 233/26, C07D 405/12, C07D 403/12, C07D 417/12, C07D 413/12, A01N 43/50, A01N 43/54

# (54) 1-Alkoxy-1,3-dizacycloalkanderivate, Verfahren zu ihrer Herstellung und ihre Verwendung als Schädlingsbekämpfungsmittel.

(30) Priorität: **12.04.91 DE 4111923**

(43) Veröffentlichungstag der Anmeldung:
**14.10.92 Patentblatt 92/42**

(45) Bekanntmachung des Hinweises auf die Patenterteilung:
**22.11.95 Patentblatt 95/47**

(84) Benannte Vertragsstaaten:
**AT BE CH DE DK ES FR GB IT LI NL PT SE**

(56) Entgegenhaltungen:
**EP-A- 0 192 060**
**EP-A- 0 292 822**

**Chemical Abstract, vol. 109, nr. 37657q**

(73) Patentinhaber: **BASF Aktiengesellschaft**
**Carl-Bosch-Strasse 38**
**D-67063 Ludwigshafen (DE)**

(72) Erfinder: **von Deyn, Wolfgang, Dr.**
**Luederitzstrasse 4**
**W-6730 Neustadt (DE)**
Erfinder: **Harreus, Albrecht, Dr.**
**Teichgasse 13**
**W-6700 Ludwigshafen (DE)**
Erfinder: **Kardorff, Uwe, Dr.**
**D 3,4**
**W-6800 Mannheim 1 (DE)**
Erfinder: **Kuenast, Christoph, Dr.**
**Salierstrasse 2**
**W-6701 Otterstadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft 1-Alkoxy-1,3-diazacycloalkanderivate der allgemeinen Formel I

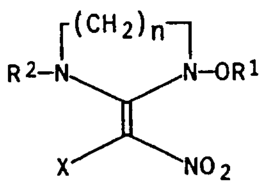

I

in der der Index und die Substitutenten folgende Bedeutung haben:

n

0 oder 1;

$R^1$

$C_1$-$C_{10}$-Alkyl, $C_1$-$C_6$-Alkylcarbonyl, $C_3$-$C_6$-Cycloalkylcarbonyl, $C_3$-$C_6$-Alkenyl oder $C_3$-$C_6$-Alkinyl, wobei diese Gruppen ein bis fünf Halogenatome und/oder einen oder zwei der folgenden Reste tragen können:

- $C_3$-$C_6$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_6$-Alkylamino, $C_1$-$C_6$-Dialkylamino, $C_1$-$C_4$-Alkylcarbonyl, $C_3$-$C_6$-Cycloalkylcarbonyl, $C_1$-$C_4$-Alkylcarbonyloxy, $C_3$-$C_6$-Cycloalkylcarbonyloxy;
- Phenylcarbonyl, Phenylcarbonyloxy, Phenyl, Phenoxy oder Phenylthio, wobei die Phenylreste ihrerseits eine bis fünf Halogenatome und/oder ein bis drei der folgenden Gruppen tragen können: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;
- einen 5- bis 6-gliedrigen heterocyclischen, aliphatischen oder aromatischen Rest, enthaltend ein bis drei Heteroatome, ausgewählt aus der Gruppe Sauerstoff, Schwefel und Stickstoff, wobei dieser Rest einen oder zwei der folgenden Substituenten tragen kann: Halogen, $C_1$-$C_4$-Alkyl, $C_3$-$C_6$-Cycloalkyl, $C_1$-$C_4$-Alkoxy und Phenyl, wobei die Phenylreste ihrerseits ein bis fünf Halogenatome und/oder ein bis drei der folgenden Gruppen tragen können: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

oder eine Gruppe -C(=O)-R, wobei

R

für Phenyl oder einen 5- bis 6-gliedrigen heterocyclischen, aliphatischen oder aromatischen Rest steht, der ein bis drei Heteroatome, ausgewählt aus der Gruppe Sauerstoff, Schwefel und Stickstoff enthält, wobei die Reste einen oder zwei der folgenden Substituenten tragen können: Cyano, Nitro, Halogen, $C_1$-$C_4$-Alkyl, $C_1$-$C_4$-Alkoxy, $C_1$-$C_4$-Halogenalkoxy, $C_1$-$C_4$-Alkylthio, $C_1$-$C_4$-Halogenalkylthio und Phenyl;

X

Wasserstoff; Halogen; $C_1$-$C_4$-Alkoxy; $C_1$-$C_4$-Alkylthio; $C_1$-$C_6$-Alkylcarbonyl; $C_1$-$C_6$-Halogenalkylcarbonyl; $C_1$-$C_6$-Alkoxycarbonyl;

$C_1$-$C_4$-Alkyl, welches ein bis neun Halogenatome und/oder ein bis drei der folgenden Gruppen tragen kann: Hydroxy, $C_1$-$C_4$-Alkoxy, $C_1$-$C_4$-Alkylthio, Phenoxy und Phenylthio;

Phenyl, Phenoxy, Phenylthio, Benzoyl oder Phenyloxycarbonyl, wobei die Phenylreste ihrerseits ein bis fünf Halogenatome und/oder ein bis drei der folgenden Gruppen tragen können: Cyano, Nitro, $C_1$-$C_4$-Alkyl und $C_1$-$C_4$-Alkoxy;

$R^2$

Wasserstoff oder $C_1$-$C_4$-Alkyl.

Außerdem betrifft die Erfindung ein Verfahren und Zwischenprodukte zur Herstellung der Verbindungen I, sie enthaltende Schädlingsbekämpfungsmittel sowie Verfahren zur Schädlingsbekämpfung.

Die Verbindungen der allgemeinen Formel I können in verschiedenen tautomeren und isomeren Formen vorliegen, die ebenfalls von der Erfindung umfaßt werden.

Aus der DE-A 24 45 421 sowie EP-A-0 292 822 und EP-A-0 192 060 sind 2-(Nitromethylen)-1,3-diazacycloalkane (beispielsweise Hexahydro-1-methyl-2-(nitromethylen)pyrimidin) mit insektizider Wirkung bekannt.

Die Wirkungshöhe bzw. Wirkungsdauer dieser vorbeschriebenen Verbindungen ist jedoch nicht immer befriedigend.

Der Erfindung lag daher die Aufgabe zugrunde, neue 1-Alkoxy-1,3-diazacycloalkanderivate mit verbesserter Wirkung bereitzustellen.

Demgemäß wurden die eingangs definierten 1-Alkoxy-1,3-diazacycloalkanderivateder allgemeinen Formel I, sowie ein Verfahren und Zwischenprodukte zu ihrer Herstellung gefunden. Ferner wurde gefunden, daß sich die Verbindungen I hervorragend zur Bekämpfung von Schädlingen eignen.

Man erhält die Verbindungen I dadurch, daß man ein 1-Alkoxyamino-2- oder 3-aminoalkan der allgemeinen Formel II in an sich bekannter Weise (J. Heterocycl. Chem. 22, 937 (1985)) in einem organischen Lösungsmittel mit einem 1,1-Bisalkylthioalken der allgemeinen Formel III umsetzt.

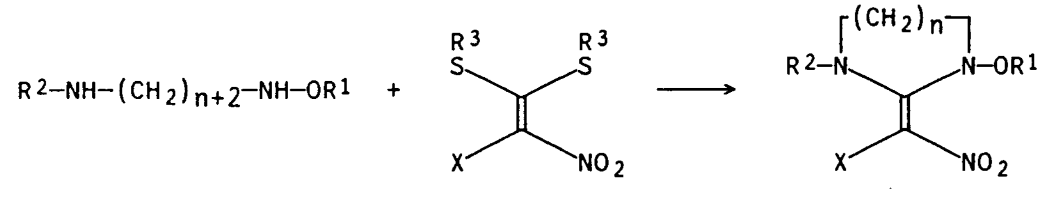

II III I

$R^3$ in der Formel III steht für eine Alkylgruppe, vorzugsweise $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere $C_1$-$C_4$-Alkyl wie Methyl, Ethyl, Propyl, 1-Methylethyl, Butyl, 1-Methylpropyl, 2-Methylpropyl und 1,1-Dimethylethyl, oder für eine Benzylgruppe.

Die Umsetzung wird üblicherweise bei Temperaturen von 0 °C bis 180 °C, vorzugsweise 20 °C bis 140 °C durchgeführt.

Als inerte organische Lösungsmittel eignen sich aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Ethanol, n-Propanol oder Isopropanol.

Es können auch Gemische der genannten Lösungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem Überschuß von 0,1 bis 10 mol-Äq., vorzugsweise 0,2 bis 1,5 mol-Äq. zu verwenden.

Man erhält die Verbindungen der allgemeinen Formel I, in denen $R^2$ $C_1$-$C_4$-Alkyl bedeutet, vorteilhaft durch Umsetzung eines 1-Alkoxy-1,3-diazacycloalkan der allgemeinen Formel Ia mit einer Verbindung der allgemeinen Formel V.

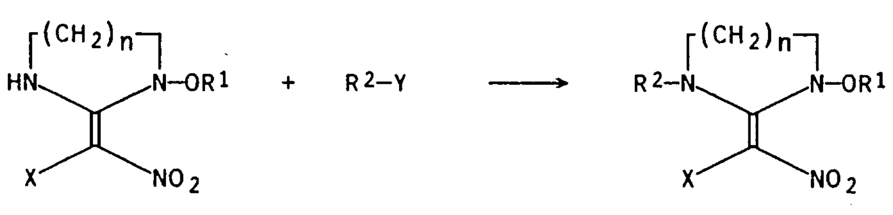

Ia ($R^2$=H) V I ($R^2$≠H)

Y in der Formel V steht für eine nucleofuge Abgangsgruppe wie Halogen, beispielsweise Fluor, Chlor, Brom und Jod, bevorzugt Chlor und Brom, oder den Rest einer Sulfonsäure wie Methansulfonyl, Trifluormethylsulfonyl, Phenylsulfonyl und p-Toluolsulfonyl, bevorzugt Methansulfonyl.

Die Umsetzung wird üblicherweise bei Temperaturen von -10 °C bis 150 °C, vorzugsweise 0 °C bis 100 °C in einem inerten organischen Lösungsmittel in Gegenwart einer Base durchgeführt.

Als inerte organische Lösungsmittel eignen sich aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Methylenchlorid, Toluol, Dimethylsulfoxid oder Dimethylformamid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Natriumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid, Kaliumhydroxid, Natriumcarbonat, Kaliumcarbonat, Natriumhydrid und Kalium-tert.-butanolat.

Die Basen werden im allgemeinen in äquimolaren Mengen eingesetzt, sie können aber auch im Überschuß oder gegebenenfalls als Lösungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem Überschuß von 0,1 bis 10 mol-Äq., vorzugsweise 0,2 bis 1,5 mol-Äq. zu verwenden.

Man erhält die Verbindungen I, in denen $R^2$ Wasserstoff und X Alkylcarbonyl, Alkoxycarbonyl, Benzoyl oder Phenyloxycarbonyl bedeutet, vorteilhaft dadurch, daß man ein 1-Alkoxy-1,3-diaza-2-methylthio-cycloalkanderivat der allgemeinen Formel VII in an sich bekannter Weise (J. Heterocyclic Chem. 17, 1413 (1980)-), gegebenenfalls in Gegenwart eines Katalysators, mit einer Nitrocarbonylverbindung der allgemeinen Formel VIII umsetzt.

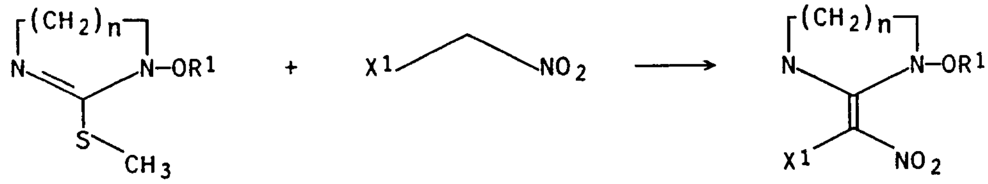

VII VIII Ic

$X^1$ in den Formeln VIII und Ic steht für $C_1$-$C_6$-Alkylcarbonyl, vorzugsweise Methylcarbonyl und Ethylcarbonyl, für $C_1$-$C_6$-Alkoxycarbonyl, vorzugsweise Methoxycarbonyl oder Ethoxycarbonyl, für Phenylcarbonyl oder für Phenyloxycarbonyl.

Die Umsetzung wird üblicherweise bei Temperaturen von 20 °C bis 180 °C, vorzugsweise 50 °C bis 150 °C durchgeführt.

Als Katalysatoren kommen allgemein Lewis-Säuren wie Bortrifluorid, Titantetrachlorid, Aluminiumtrichlorid und Zinkchlorid, besonders bevorzugt Zinkchlorid in Betracht.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem Überschuß von 0,1 bis 10 mol-Äq., vorzugsweise 0,2 bis 1,5 mol-Äq. zu verwenden.

Die für die Umsetzung benötigten 1-Alkoxy-1,3-diaza-2-methylthio-cycloalkanderivate der allgemeinen Formel VII lassen sich nach allgemein bekannten chemischen Verfahren (J.Am.Chem.Soc. 80, 3339 (1950)) aus den 1-Alkoxyamino-2- oder -3-aminoalkanen der allgemeinen Formel II herstellen.

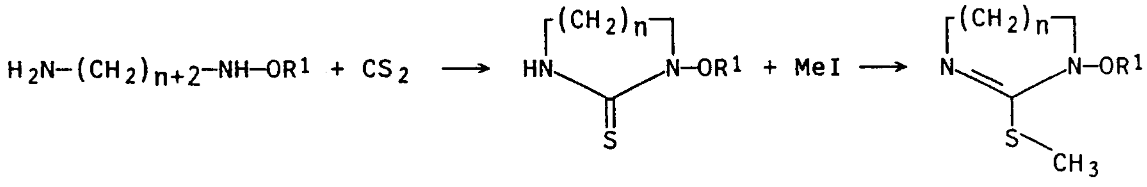

II ($R^2$=H) VIIa VII

Die Nitroalkylverbindungen der allgemeinen Formel VIII sind bekannt oder nach bekannten Methoden herstellbar (Tetrahedron Lett. 27, 493 (1986)).

Außerdem erhält man weitere Verbindungen der allgemeinen Formel I, in der $R^2$ für Wasserstoff steht, vorteilhaft in an sich bekannter Weise aus 1-Alkoxy-1,3-diazacycloalkanen der allgemeinen Formel Ib durch Substitution des α-Kohlenstoffatoms der Nitromethylengruppe.

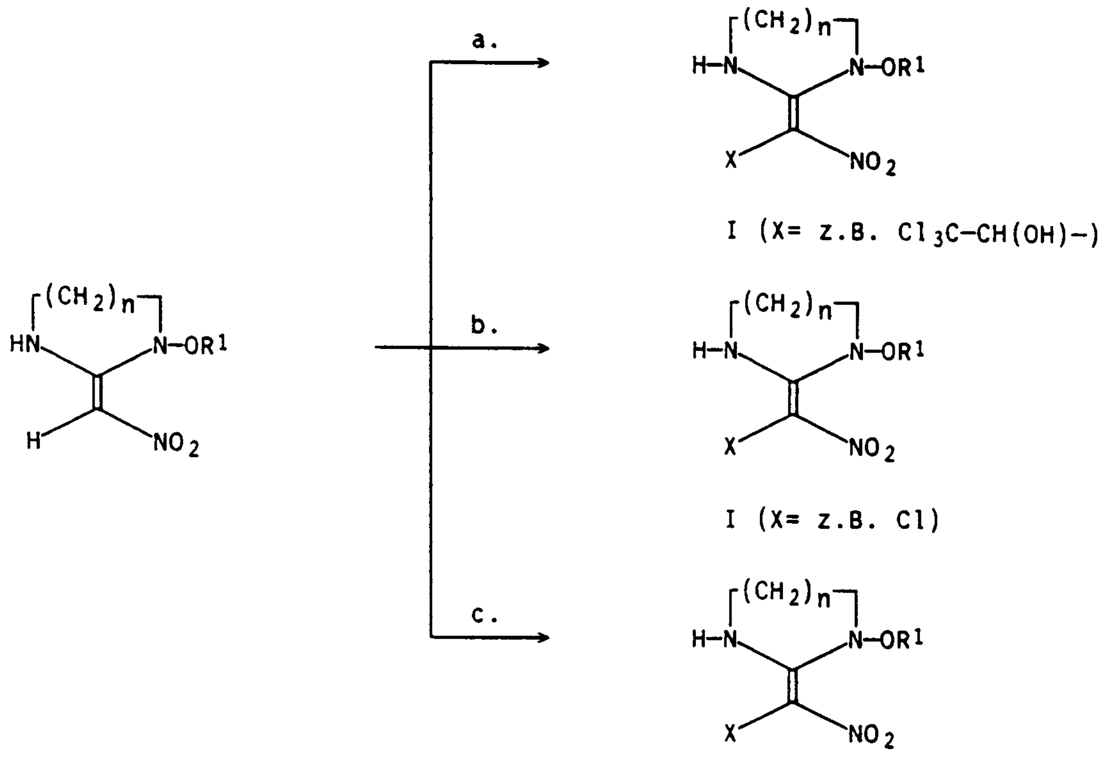


I (X= z.B. $Cl_3C-CH(OH)-$)

I (X= z.B. Cl)

I (X= z.B. Phenylthio)

a. Beispielsweise können an das α-Kohlenstoffatom der Nitromethylengruppe aktive Aldehyde wie Formaldehyd oder Chloral addiert werden (US-A 4,033,952 und EP-A 292 822).

b. Darüber hinaus kann das alpha-Kohlenstoffatom auch durch Halogenierungsmittel wie N-Chlorsuccinimid, N-Bromsuccinimid und Halogen an sich halogeniert werden (US-A 3,962,233).

c. Weiterhin kann das alpha-Kohlenstoffatom der Nitromethylengruppe acyliert oder sulfinyliert werden (US-A 4,076,813 und US-A 3,969,354).

Die für die Synthese der Verbindungen I benötigten 1-Alkoxyamino-2-aminoalkane der allgemeinen Formel II, in denen n den wert 0 hat, sind bekannt oder nach bekannten Methoden herstellbar (DE-A 27 09 720).

Beispielsweise ist die Herstellung von derartigen 1-Alkoxyamino-ω-aminoalkanen der allgemeinen Formel II, deren Substituent $R^1$ für substituiertes Benzyl steht, in Pol.J.Chem., 55(5), 1163-7 (1981) beschrieben.

1-Alkoxyamino-3-aminoalkane der allgemeinen Formel IIa sind mit Ausnahme der Verbindungen, in denen $R^1$ Benzyl oder substituiertes Benzyl bedeutet (Chem. Abstracts, Band 109, Nr. 37657q (1988)), neu

und Teil der vorliegenden Erfindung.

Man erhält diese neuen Zwischenprodukte dadurch, daß man ein Alkylamin der allgemeinen Formel IIb in an sich bekannter Weise in einem inerten organischen Lösungsmittel mit einem Hydroxylamin der allgemeinen Formel IV umsetzt und anschließend die Schutzgruppe abspaltet.

$$Y-(CH_2)_2-N(R^2)-T^1 + T^2-NH-OR^1 \rightarrow R^2N(T^1)-(CH_2)_3-N(T^2)OR^1 \rightarrow R^2NH-(CH_2)_3-NHOR^1$$

IIb IV IId IIa

In der Formel IIb steht Y für eine Abgangsgruppe wie Sulfonsäurereste oder Halogenatome. Unter den Sulfonsäureresten sind Methansulfonyl, Trifluormethansulfonyl, Benzolsulfonyl und p-Toluolsulfonyl, unter den Halogenatomen sind Chlor und Brom bevorzugt.

$T^2$ in der Formel IV steht für Wasserstoff oder für eine Schutzgruppe für Aminofunktionen und $T^1$ steht für eine Schutzgruppe für Aminofunktionen wie beispielsweise in T.W. Greene, Protective Groups in Organic Synthesis, Chapter 7, Wiley, 1981 beschrieben, besonders bevorzugte Schutzgruppen sind Methoxycarbonyl, Ethoxycarbonyl, tert.-Butoxycarbonyl, 2,2,2-Trichlorethoxycarbonyl und Benzyloxycarbonyl.

Außerdem erhält man die Zwischenprodukte der allgemeinen Formel IIa, in denen $R^2$ Wasserstoff bedeutet, dadurch, daß man ein Alkylamin der allgemeinen Formel IIe' mit einem Hydroxylamin der allgemeinen Formel IV wie oben angegeben umsetzt.

$$Y-(CH_2)_3-N=T^3 + T^2NHOR^1 \longrightarrow T^3N-C(CH_2)_3-NT^2OR^1 \longrightarrow H_2N-C(CH_2)_3-NHOR^1$$

IIe' IV IIf IIa

$T^3$ in der Formel IIe' steht für eine zweizähnige, cyclische Schutzgruppe für Aminofunktionen, beispielsweise die Phthalimidgruppe.

Diese Umsetzung erfolgt üblicherweise bei Temperaturen von -20 °C bis 180 °C, vorzugsweise 0 °C bis 120 °C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Methanol, Ethanol, n-Propanol, Isopropanol, n-Butanol, Tetrahydrofuran und Dimethylformamid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Natriumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid, Kaliumhydroxid, Natriumhydrogencarbonat, Natriumhydrid und Kalium-tert.-butanolat.

Die Edukte werden im allgemeinen in äquimolaren Mengen miteinander umgesetzt. Es kann, beispielsweise zur Steigerung der Ausbeute, vorteilhaft sein, einen der Ausgangsstoffe in einem Überschuß von 0,1 bis 10 mol-Äq., vorzugsweise 0,2 bis 1,5 mol-Äq. zu verwenden.

Man erhält die neuen Zwischenprodukte IIa beispielsweise auch dadurch, daß man ein 1-Amino-3-hydroxyaminopropan der allgemeinen Formel IIc in an sich bekannter Weise in einem inerten organischen

Lösungsmittel in Gegenwart einer Base mit einem Alkylierungsmittel der allgemeinen Formel VI umsetzt.

$$\underset{IIc}{T^1\text{-NH-}(CH_2)_3\text{-N(OH)-}T^1} + \underset{VI}{Y\text{-}R^1} \rightarrow \underset{IId}{R^2N(T^1)\text{-}(CH_2)_3\text{-N}(T^1)OR^1} \rightarrow \underset{IIa}{R^2NH\text{-}(CH_2)_3\text{-NHOR}^1}$$

$T^1$ in der Formel IIc hat im allgemeinen und im besonderen die vorstehend gegebene Bedeutung.

In der Formel VI steht Y für eine nucleofuge Abgangsgruppe wie vorstehend bei der Formel IIb im allgemeinen und im besonderen genannt.

Diese Umsetzung erfolgt üblicherweise bei Temperaturen von -20 °C bis 180 °C, vorzugsweise 0 °C bis 120 °C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Toluol, Tetrahydrofuran und Dimethylformamid.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Natriumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid, Kaliumhydroxid, Natriumhydrogencarbonat, Natriumhydrid und Kalium-tert.-butanolat.

Die Edukte werden im allgemeinen in äquimolaren Mengen miteinander umgesetzt. Es kann, beispielsweise zur Steigerung der Ausbeute, vorteilhaft sein, einen der Ausgangsstoffe in einem Überschuß von 0,1 bis 10 mol-Äq., vorzugsweise 0,2 bis 1,5 mol-Äq. zu verwenden.

1-Amino-3-hydroxyaminopropan Dihydrochlorid ist in Biochemistry 10, 4894 (1971) beschrieben.

Die Schutzgruppen werden nach den bei Greene (Protective Groups in Organic Synthesis, Chapter 7, Wiley (1981)) aufgeführten Methoden abgespalten.

Die 1,1-Bisalkylthioalkene der Formel III sind zum Teil in Angew. Chem. 100, 591-604 (1967) beschrieben oder können nach den dort beschriebenen Methoden hergestellt werden.

Werden die Verbindungen der Formel II in Form ihrer Säureadditionssalze eingesetzt, wird die Umsetzung zweckmäßigerweise unter Zusatz eines säurebindenden Mittels durchgeführt. Man verwendet normalerweise mindestens äquivalente Mengen des säurebindenden Mittels, kann dieses aber auch im Überschuß oder gegebenenfalls als Lösungsmittel einsetzen.

Als säurebindende Mittel eignen sich beispielsweise Hydroxide von Alkali- und Erdalkalimetallen wie Natriumhydroxid, Kaliumhydroxid und Calciumhydroxid; Alkoholate von Alkali- und Erdalkalimetallen wie Natriummethylat, Natriumethylat, Calciummethanolat oder Kalium-tert.-butylat; Alkali- oder Erdalkalimetallhydride wie Natriumhydrid, Kaliumhydrid oder Calciumhydrid; Alkali- und Erdalkalicarbonate wie Natriumcarbonat, Kaliumcarbonat oder Calciumcarbonat; aliphatische, aromatische oder heterocyclische Amine wie Dimethylamin, Triethylamin, Diisopropylamin, Piperidin, Piperazin, Pyrrolidin, 1.5-Diazabicyclo[4,3,0]-non-5-en (DBN), 1,8-Diazabicyclo-[5,4,0]-undec-7-en (DBU), 1,4-Diazabicyclo-[2,2,2]-octan (DABCO), Pyridin oder Pyrrol.

Die für die Umsetzung benötigten Hydroxylaminderivate IV, in denen $T^2$ für Wasserstoff steht, sind bekannt oder lassen sich nach bekannten Methoden herstellen (DE-A 38 38 310).

Die für die Umsetzung benötigten Alkylderivate der Formeln V und VI sind bekannt, kommerziell erhältlich oder lassen sich nach allgemein bekannten chemischen Verfahren herstellen.

Im Hinblick auf die bestimmungsgemäße Verwendung der Alkoxy-1,3-diazacycloalkanderivate der allgemeinen Formel I kommen als Substituenten folgende Reste in Betracht:

$R^1$

$C_1$-$C_{10}$-Alkyl, besonders $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere $C_1$-$C_4$-Alkyl;

$C_1$-$C_6$-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise $C_1$-$C_4$-Alkylcarbonyl, insbesondere $C_1$-$C_2$-Alkylcarbonyl;

$C_3$-$C_6$-Cycloalkylcarbonyl wie Cyclopropylcarbonyl, Cyclobutylcarbonyl, Cyclopentylcarbonyl und Cyclohexylcarbonyl, vorzugsweise Cyclopropylcarbonyl, Cyclopentylcarbonyl und Cyclohexylcarbonyl;

$C_3$-$C_6$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl und 2-Butenyl;

oder $C_3$-$C_6$-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Hethyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, insbesondere 2-Propinyl und 2-Butinyl;

wobei diese Gruppen ein bis fünf Halogenatome wie Fluor, Chlor, Brom und Iod, vorzugsweise Fluor, Chlor und Brom, insbesondere Fluor und Chlor,

und/oder einen oder zwei der folgenden Reste tragen können:

- $C_3$-$C_6$-Cycloalkyl wie Cyclopropyl, Cyclobutyl, Cyclopentyl, Cyclohexyl, Cycloheptyl und Cyclooctyl, insbesondere Cyclopropyl und Cyclohexyl;
- $C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere $C_1$-$C_2$-Alkoxy;
- $C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere 2,2,2-Trifluorethyloxy und 2-Chlor-2,2-difluorethoxy;
- $C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, insbesondere Methylthio;
- $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor2-fluorethylthio, 2-Chlor-2,2-difluorethylthio, 2,2-Dichlor-2-fluorethylthio, 2,2,2-Trichlorethylthio und Pentafluorethylthio, insbesondere Trichlormethylthio;

- $C_1$-$C_6$-Alkylamino wie Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino und 1-Ethyl-2-methylpropylamino, insbesondere $C_1$-$C_2$-Alkylamino;
- Di-$C_1$-$C_6$-alkylamino, besonders Di-$C_1$-$C_4$-alkylamino wie N,N-Dimethylamino, N,N-Diethylamino, N,N-Dipropylamino, N,N-Di-(1-methylethyl)-amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)-amino, N-Butyl-N-methylamino, N-Methyl-N-(1-methylpropyl)-amino, N-Methyl-N-(2-methylpropyl)amino, N-(1,1-Dimethylethyl)-N-methylamino, N-Ethyl-N-propylamino, N-Ethyl-N-(1-methyl-ethyl)amino, N-Butyl-N-ethylamino, N-Ethyl-N-(1-methyl-propyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methyl-ethyl)amino, N-(1-Methylethyl)-N-(1-methylpropyl)-amino, N-(1-Methylethyl)-N-(2-methylpropyl)amino, N-(1,1-Di-methylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methyl-propyl)amino, N-(1,1-Dimethylethyl)-N-(1-methylpropyl)amino und N-(1,1-Dimethylethyl)-N-(2-methylpropyl)amino, insbesondere N,N-Dimethylamino und N,N-Diethylamino;
- $C_1$-$C_6$-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, , Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise $C_1$-$C_4$-Alkylcarbonyl, insbesondere $C_1$-$C_2$-Alkylcarbonyl;
- $C_3$-$C_6$-Cycloalkylcarbonyl wie Cyclopropylcarbonyl, Cyclobutylcarbonyl, Cyclopentylcarbonyl und Cyclohexylcarbonyl, vorzugsweise Cyclopropylcarbonyl, Cyclopentylcarbonyl und Cyclohexylcarbonyl;
- $C_1$-$C_6$-Alkylcarbonyloxy wie Methylcarbonyloxy, Ethylcarbonyloxy, Propylcarbonyloxy, 1-Methylethylcarbonyloxy, Butylcarbonyloxy, 1-Methylpropylcarbonyloxy, 2-Methylpropylcarbonyloxy, 1,1-Dimethylethylcarbonyloxy, Pentylcarbonyloxy, 1-Methylbutylcarbonyloxy, 2-Methylbutylcarbonyloxy, 3-Methylbutylcarbonyloxy, 1,1-Dimethylpropylcarbonyloxy, 1,2-Dimethylpropylcarbonyloxy, 2,2-Dimethylpropylcarbonyloxy, 1-Ethylpropylcarbonyloxy, Hexylcarbonyloxy, 1-Methylpentylcarbonyloxy, 2-Methylpentylcarbonyloxy, 3-Methylpentylcarbonyloxy, 4-Methylpentylcarbonyloxy, 1,1-Dimethylbutylcarbonyloxy, 1,2-Dimethylbutylcarbonyloxy, 1,3-Dimethylbutylcarbonyloxy, 2,2-Dimethylbutylcarbonyloxy, 2,3-Dimethylbutylcarbonyloxy, 3,3-Dimethylbutylcarbonyloxy, 1-Ethylbutylcarbonyloxy, 2-Ethylbutylcarbonyloxy, 1,1,2-Trimethylpropylcarbonyloxy, 1,2,2-Trimethylpropylcarbonyloxy, 1-Ethyl-1-methylpropylcarbonyloxy und 1-Ethyl-2-methylpropylcarbonyloxy, vorzugsweise $C_1$-$C_4$-Alkylcarbonyloxy, insbesondere $C_1$-$C_2$-Alkylcarbonyloxy;
- $C_3$-$C_6$-Cycloalkylcarbonyloxy wie Cyclopropylcarbonyloxy, Cyclobutylcarbonyloxy, Cyclopentylcarbonyloxy und Cyclohexylcarbonyloxy, vorzugsweise Cyclopropylcarbonyloxy, Cyclopentylcarbonyloxy und Cyclohexylcarbonyloxy;
- Phenylcarbonyl, Phenylcarbonyloxy, Phenyl, Phenoxy oder Phenylthio, wobei die Phenylreste ihrerseits eine bis fünf Halogenatome wie Fluor, Chlor, Brom und Iod, insbesondere Fluor und Chlor und/oder ein bis drei der folgenden Gruppen tragen können:

  Cyano, Nitro,

  $C_1$-$C_4$-Alkyl wie Methyl, Ethyl, Propyl, 1-Methylethyl, Butyl, 1-Methylpropyl, 2-Methylpropyl und 1,1-Dimethylethyl, insbesondere Methyl; $C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;

  $C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere $C_1$-$C_2$-Alkoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethoxy;

$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, insbesondere Methylthio;

$C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor2-fluorethylthio, 2-Chlor-2,2-difluorethylthio, 2,2-Dichlor-2-fluorethylthio, 2,2,2-Trichlorethylthio und Pentafluorethylthio, insbesondere Trichlormethylthio und Trifluormethylthio;

- einen 5- bis 6-gliedrigen heterocyclischen, aliphatischen oder aromatischen Rest, enthaltend ein bis drei Heteroatome, ausgewählt aus der Gruppe Sauerstoff, Schwefel und Stickstoff, zum Beispiel fünfring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl,1,2,4-Triazol-3-yl, 1,3,4-Oxadiazol-2-yl, 1,3,4-Thiadiazol-2-yl und 1,3,4-Triazol-2-yl vorzugsweise 5-Isoxazolyl, 2-Thienyl und 5-Pyrazolyl, insbesondere 5-Thiazolyl und 5-Isoxazolyl;

sechsring Heteroaromaten enthaltend ein bis drei Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, insbesondere 5-Pyrimidinyl, 2-Pyridyl und 3-Pyridyl;

5- bis 6-gliedrige, gesättigte oder teilweise ungesättigte Heterocyclen, enthaltend ein bis drei Stickstoffatome und/oder ein oder zwei Sauerstoff-oder Schwefelatom wie 2-Tetrahydrofuranyl, 3-Tetrahydrofuranyl, 2-Tetrahydrothienyl, 3-Tetrahydrothienyl, 1-Pyrrolidinyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl; 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Thiazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydrothien-2-yl, 2,3-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 4,5-Isoxazolin-3-yl, 2,3-Isoxazolin-4-yl, 4,5-Isoxazolin-4-yl, 2,3-Isoxazolin-5-yl, 4,5-Isoxazolin-5-yl, 2,3-Isothiazolin-3-yl, 4,5-Isothiazolin-3-yl, 2,3-Isothiazolin-4-yl, 4,5-Isothiazolin-4-yl, 2,3-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 1,3-Dioxolan-2-yl, 1,3-Dioxolan-4-yl, 1,3-Oioxan-5-yl, 1,4-Dioxan-2-yl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, insbesondere 2-Tetrahydrofuranyl, 1,3-Dioxolan-2-yl und 1,3-Dioxan-2-yl,

wobei diese Reste einen oder zwei der folgenden Substituenten tragen können: Halogen wie Fluor, Chlor, Brom und Iod, insbesondere Fluor und Chlor;

$C_1$-$C_4$-Alkyl wie Methyl, Ethyl, Propyl, 1-Methylethyl, Butyl, 1-Methylpropyl, 2-Methylpropyl und 1,1-Dimethylethyl, insbesondere $C_1$-$C_2$-Alkyl;

$C_3$-$C_6$-Cycloalkyl wie Cyclopropyl, Cyclobutyl, Cyclopentyl und Cyclohexyl, vorzugsweise Cyclopropyl, Cyclopentyl und Cyclohexyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere $C_1$-$C_2$-Alkoxy,

und Phenyl; wobei die Phenylreste ihrerseits ein bis fünf Halogenatome wie Fluor, Chlor, Brom und Jod, vorzugsweise Fluor und Chlor, und/oder ein bis drei der folgenden Gruppen tragen können:

Cyano, Nitro,

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise $C_1$-$C_2$-Alkyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Difluormethyl und Trifluormethyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise $C_1$-$C_2$-Alkoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Difluormethyloxy;

$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise $C_1$-$C_2$-Alkylthio;

und $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;

oder eine Gruppe -C(=O)-R, wobei

R

für Phenyl oder einen der vorstehend im allgemeinen und im besonderen genannten 5- bis 6-gliedrigen heterocyclischen, aliphatischen oder aromatischen Reste steht, der ein bis drei Heteroatome, ausgewählt aus der Gruppe Sauerstoff, Schwefel und Stickstoff enthält, und wobei diese Reste einen oder zwei der folgenden Substituenten tragen können: Cyano, Nitro, Halogen wie Fluor, Chlor, Brom und Jod, vorzugsweise Fluor und Chlor,

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise $C_1$-$C_2$-Alkyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Difluormethyl und Trifluormethyl;

$C_1$-$C_4$-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise $C_1$-$C_2$-Alkoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Difluormethyloxy;

$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise $C_1$-$C_2$-Alkylthio;

$C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;

und Phenyl;

X

Wasserstoff;

Halogen wie Fluor, Chlor, Brom und Iod, insbesondere Chlor und Brom;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere Methoxy;

$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, insbesondere Methylthio;

$C_1$-$C_6$-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, insbesondere $C_1$-$C_2$-Alkylcarbonyl;

$C_1$-$C_6$-Halogenalkylcarbonyl, besonders $C_1$-$C_2$-Halogenalkylcarbonyl wie Chlormethylcarbonyl, Dichlormethylcarbonyl, Trichlormethylcarbonyl, Fluormethylcarbonyl, Difluormethylcarbonyl, Trifluormethylcarbonyl, Chlorfluormethylcarbonyl, Dichlorfluormethylcarbonyl, Chlordifluormethylcarbonyl, 1-Fluorethylcarbonyl, 2-Fluorethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl und Pentafluorethylcarbonyl, insbesondere Trichlormethylcarbonyl und Trifluormethylcarbonyl;

$C_1$-$C_6$-Alkoxycarbonyl wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl, 1,1-Dimethylethoxycarbonyl, Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethylpropoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl, vorzugsweise $C_1$-$C_2$-Alkoxycarbonyl;

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise $C_1$-$C_2$-Alkyl,

welches ein bis neun Halogenatome wie Fluor, Chlor, Brom und Iod, vorzugsweise Fluor und Chlor,

und/oder ein bis drei der folgenden Gruppen tragen kann: Hydroxy,

$C_1$-$C_4$-Alkoxy wie Methoxy, Ethoxy, Propyloxy, 1-Methylethoxy, Butyloxy,
1-Methyl-propyloxy, 2-Methylpropyloxy und 1,1-Dimethylethoxy, insbesondere $C_1$-$C_2$-Alkoxy;

$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, insbesondere Methylthio;

Phenoxy und Phenylthio;

Phenyl, Phenoxy, Phenylthio, Benzoyl oder Phenyloxycarbonyl, wobei die Phenylreste ihrerseits ein bis fünf Halogenatome wie Fluor, Chlor, Brom und Iod, insbesondere Fluor und Chlor,

und/oder ein bis drei der folgenden Gruppen tragen können:

Cyano, Nitro,

$C_1$-$C_4$-Alkyl wie Methyl, Ethyl, Propyl, 1-Methylethyl, Butyl, 1-Methylpropyl, 2-Methylpropyl und 1,1-Dimethylethyl, insbesondere $C_1$-$C_2$-Alkyl,

und $C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, insbesondere Methoxy;

$R^2$

Wasserstoff

oder $C_1$-$C_4$-Alkyl wie Methyl, Ethyl, Propyl, 1-Methylethyl, Butyl, 1-Methylpropyl, 2-Methylpropyl und 1,1-Dimethylethyl, insbesondere Methyl.

Der Index n bedeutet 0 oder 1.

Außerdem werden 1-Alkoxy-1,3-diazaycloalkanderivate der allgemeinen Formel I bevorzugt, in der der Index n 1 bedeutet und die Reste X und $R^2$ für Wasserstoff stehen.

Im Hinblick auf die bestimmungsgemäße Verwendung der 1-Alkoxyamino-3-aminoalkanderivate der allgemeinen Formel IIa als Zwischenprodukte kommen als Substituenten folgende Reste in Betracht:

$R^1$

$C_1$-$C_6$-Alkyl wie vorstehend im allgemeinen und im besonderen genannt, wobei diese Gruppen ein bis fünf Halogenatome wie vorstehend im allgemeinen und im besonderen genannt, und/oder einen oder zwei der vorstehend im allgemeinen und im besonderen genannten folgenden Reste tragen können: $C_3$-$C_6$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_6$-Alkylamino, $C_1$-$C_6$-Dialkylamino, $C_1$-$C_4$-Alkylcarbonyl, $C_3$-$C_6$-Cycloalkylcarbonyl, $C_1$-$C_4$-Alkylcarbonyloxy, $C_3$-$C_6$-Cycloalkylcarbonyloxy, Benzoyl, Benzoyloxy, Phenyl, Phenoxy oder Phenylthio, wobei die Phenylreste ihrerseits eine bis fünf Halogenatome und/oder ein bis drei der folgenden Gruppen tragen können: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio, wobei $R^1$ nicht für Benzyl oder substituiertes Benzyl steht;

$C_1$-$C_6$-Alkylcarbonyl oder $C_3$-$C_6$-Cycloalkylcarbonyl wie vorstehend im allgemeinen und im besonderen genannt, wobei diese Gruppen ein bis fünf Halogenatome, wie vorstehend im allgemeinen und im besonderen genannt, und/oder einen oder zwei der vorstehend im allgemeinen und im besonderen

genannten folgenden Reste tragen können: $C_3$-$C_6$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_6$-Alkylamino, $C_1$-$C_6$-Dialkylamino, Phenyl, Phenoxy oder Phenylthio, wobei die Phenylreste ihrerseits ein bis fünf Halogenatome und/oder ein bis drei der folgenden Gruppen tragen können: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

$C_3$-$C_6$-Alkenyl oder $C_3$-$C_6$-Alkinyl wie vorstehend im allgemeinen und im besonderen genannt, wobei diese Gruppen ein bis fünf Halogenatome wie vorstehend im allgemeinen und im besonderen genannt, und/oder einen oder zwei der vorstehend im allgemeinen und im besonderen genannten folgenden Reste tragen können: $C_3$-$C_6$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_6$-Alkylamino, $C_1$-$C_6$-Dialkylamino, $C_1$-$C_4$-Alkylcarbonyl, $C_3$-$C_6$-Cycloalkylcarbonyl, $C_1$-$C_4$-Alkylcarbonyloxy, $C_3$-$C_6$-Cycloalkylcarbonyloxy, Benzoyl, Benzoyloxy, Phenyl, Phenoxy oder Phenylthio, wobei die Phenylreste ihrerseits eine bis fünf Halogenatome und/oder ein bis drei der folgenden Gruppen tragen können: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

einen 5- bis 6-gliedrigen heterocyclischen, aliphatischen oder aromatischen Rest, enthaltend ein bis drei Heteroatome, ausgewählt aus der Gruppe Sauerstoff, Schwefel und Stickstoff, wie vorstehend im allgemeinen und im besonderen genannt, wobei dieser Rest einen oder zwei der vorstehend im allgemeinen und im besonderen genannten folgenden Substituenten tragen kann: Halogen, $C_1$-$C_4$-Alkyl, $C_3$-$C_6$-Cycloalkyl, $C_1$-$C_4$-Alkoxy und Phenyl, wobei die Phenylreste ihrerseits ein bis fünf Halogenatome wie vorstehend im allgemeinen und im besonderen genannt, und/oder ein bis drei der vorstehend im allgemeinen und im besonderen genannten folgenden Gruppen tragen können: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

oder eine Gruppe -C(=O)-R, wobei

R

für Phenyl oder einen der vorstehend im allgemeinen und im besonderen genannten 5- bis 6-gliedrigen heterocyclischen, aliphatischen oder aromatischen Reste steht, der ein bis drei Heteroatome, ausgewählt aus der Gruppe Sauerstoff, Schwefel und Stickstoff enthält, und wobei diese Reste einen oder zwei der vorstehend im allgemeinen und im besonderen genannten folgenden Substituenten tragen können: Cyano, Nitro, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio und Phenyl;

$R^2$

Wasserstoff oder $C_1$-$C_4$-Alkyl wie vorstehend im allgemeinen und im besonderen genannt.

Beispiele für besonders bevorzugte Verbindungen der allgemeinen Formel I

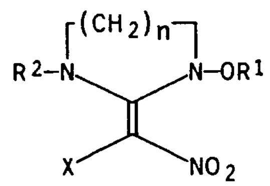

I

sind in der folgenden Tabelle zusammengestellt.

**Tabelle**

| $R^1$ | X | n | $R^2$ |
|---|---|---|---|
| $-CH_2-CH_2-CH_3$ | H | 1 | H |
| $-CH(CH_3)_2$ | H | 1 | H |
| $-CH(CH_3)_2$ | H | 1 | $CH_3$ |
| $-CH_2-(CH_2)_3-CH_3$ | H | 1 | H |
| $-CH(C_2H_5)(CH_3)$ | H | 1 | H |
| $-CH_2-CH-(CH_3)_2$ | H | 1 | H |
| $-C(CH_3)_3$ | H | 1 | H |
| $-CH_2-CH=CH_2$ | H | 1 | H |
| $-CH_2-CH=CH_2$ | H | 1 | $CH_3$ |
| $-CH_2-CH=CH_2$ | H | 1 | $C_2H_5$ |
| $-CH_2-CH=CH-CH_3$ | H | 1 | H |
| $-CH_2-CH=CH-(CH_2)_2-CH_3$ | H | 1 | H |
| $-CH_2-CH_2-C(=CH_2)(CH_3)$ | H | 1 | H |
| $-CH(CH_3)-CH=CH_2$ | H | 1 | H |
| $-CH_2-CH_2-CH=CH_2$ | H | 1 | H |
| $-CH_2-C\equiv CH$ | H | 1 | H |
| $-CH_2-C\equiv C-CH_3$ | H | 1 | H |
| $-CH_2-C\equiv C-CH_3$ | H | 1 | $CH_3$ |
| $-CH(CH_3)-C\equiv CH$ | H | 1 | H |
| $-CH_2-CBr_3$ | H | 1 | H |
| $-CH_2-CCl_3$ | H | 1 | H |
| $-CH_2-CH_2-Br$ | H | 1 | H |
| $-CH_2-CF_2Br$ | H | 1 | H |
| $-CH_2-CF_2Cl$ | H | 1 | H |

Tabelle (Forts.)

| $R^1$ | X | n | $R^2$ |
|---|---|---|---|
| $-CH_2-CF_3$ | H | 1 | H |
| $-CH_2-CF_3$ | H | 1 | $CH_3$ |
| $-CH_2-CH_2-CH(Cl)-CH_2Cl$ | H | 1 | H |
| $-CH(CH_2Cl)CH=CH_2$ | H | 1 | H |
| $-CH_2-CH=CH_2-CH_2Cl$ | H | 1 | H |
| $-CH_2-C(Cl)=CH_2$ | H | 1 | H |
| $-CH_2-C(Cl)=CH_2$ | H | 1 | $CH_3$ |
| $-CH_2-C(CH_3)(CH_2Cl)_2$ | H | 1 | H |
| $-CH_2-CH_2Cl$ | H | 1 | H |
| $-CH_2C(CH_3)_2(CH_2Cl)$ | H | 1 | H |
| $-CH_2-CH_2CF=CF_2$ | H | 1 | H |
| $-CH_2$-Cyclopropyl | H | 1 | H |
| $-CH_2$-Cyclopropyl | H | 1 | $CH_3$ |
| $-CH_2$-Cyclopentyl | H | 1 | H |
| $-CH_2$-Cyclohexyl | H | 1 | H |
| $-CH_2-O-CH_2-CH_3$ | H | 1 | H |
| $-CH_2-O-CH_3$ | H | 1 | H |
| $-CH_2-CH_2-O-CH_3$ | H | 1 | H |
| $-CH_2-CH_2-O-C_2H_5$ | H | 1 | H |
| $-CH_2-CH_2-O-C_2H_5$ | H | 1 | $CH_3$ |
| $-C(CH_3)_2-O-CH_3$ | H | 1 | H |
| $-CH_2CH_2-O-CH_2-CH_2Cl$ | H | 1 | H |
| $-CH_2-CH_2-O-CH_2-CF_3$ | H | 1 | H |
| $-CH_2-(CH_2)_2-O-CH_3$ | H | 1 | H |

Tabelle (Forts.)

| $R^1$ | X | n | $R^2$ |
|---|---|---|---|
| $-CH_2-CH_2-CH=CH-CH_2-O-CH_3$ | H | 1 | H |
| $-CH_2-CH_2-N(CH_3)_2$ | H | 1 | H |
| $-CH_2-CH_2-CH_2-N(C_2H_5)_2$ | H | 1 | H |
| $-CH_2-CH_2-S-CH_3$ | H | 1 | H |
| $-CH_2-CH_2-S-C(CH_3)_3$ | H | 1 | H |
| $-CH_2-CH_2-S-CHF_2$ | H | 1 | H |
| $-CH_2$-Phenyl | H | 1 | H |
| $-CH_2$-Phenyl | H | 1 | $CH_3$ |
| $-CH_2$-Phenyl | H | 1 | $C_2H_5$ |
| $-CH_2$-Phenyl | H | 1 | $CH(CH_3)_2$ |
| $-CH_2$-[4-Cl-Phenyl] | H | 1 | H |
| $-CH_2$-[3-Cl-Phenyl] | H | 1 | H |
| $-CH_2$-[2,4-$Cl_2$-Phenyl] | H | 1 | H |
| $-CH_2$-[3-$CF_3$-Phenyl] | H | 1 | H |
| $-CH_2$-[4-($OCF_3$)-Phenyl] | H | 1 | H |
| $-CH_2$-[2-($OCH_3$)-Phenyl] | H | 1 | H |
| $-CH_2$-[2,4,6-$(CH_3)_3$-Phenyl] | H | 1 | H |
| $-CH_2$-[4=($SCH_3$)-Phenyl] | H | 1 | H |
| $-CH_2-CH=CH-CH_2$-[4-F-Phenyl] | H | 1 | H |
| $-CH_2-CH=CH-CH_2$-[4-($C(CH_3)_3$)-Phenyl] | H | 1 | H |
| $-CH_2-(CH_2)_3$-Phenyl | H | 1 | H |
| $-CH_2-CH_2$-Phenyl | H | 1 | H |
| $-CH_2-C{\equiv}C-CH_2$-Phenyl | H | 1 | H |
| $-CH_2-(CH_2)_2$-O-Phenyl | H | 1 | H |

Tabelle (Forts.)

| $R^1$ | X | n | $R^2$ |
|---|---|---|---|
| $-CH_2-CH(OCH_2CH_3)_2$ | H | 1 | H |
| $-CH_2-CH(OCH_3)_2$ | H | 1 | H |
| $-CH_2-C(OCH_3)_2CH_3$ | H | 1 | H |
| $-CH_2-CH=CH-$Phenyl | H | 1 | H |
| $-CH_2-CH_2-O-$Phenyl | H | 1 | H |
| $-CH_2$ 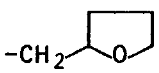 | H | 1 | H |
| $-CH_2-CH_2-$[4-$NO_2$-Phenyl] | H | 1 | H |
| $-CH_2-(CH_2)_8-CH_3$ | H | 1 | H |
| $-CH_2-(CH_2)_2-$Phenyl | H | 1 | H |
| $-CH_2-C(Br)=CH_2$ | H | 1 | H |
| $-CH_2-C(CH_3)_3$ | H | 1 | H |
| $-CH_2-CH(CH_3)-$Phenyl | H | 1 | H |
| $-CH_2-CH_2-O-$[4-F-Phenyl] | H | 1 | H |
| $-CH_2-(CH_2)_3-O-$Phenyl | H | 1 | H |
| $-CH_2-CH=C(CH_3)_2$ | H | 1 | H |
| $-CH_2-CH_2$ 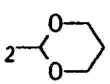 | H | 1 | H |
| $-CH_2-CH_2-O-$[2-Cl-Phenyl] | H | 1 | H |
| $-CH_2-CH_2-O-$[4-Cl-Phenyl] | H | 1 | H |

Tabelle (Forts.)

| $R^1$ | X | n | $R^2$ |
|---|---|---|---|
| $-CH_2-CH_2-O-[2,6-Cl_2-Phenyl]$ | H | 1 | H |
| $-CH_2-CH_2-O-[2-CH_3,4-Cl-Phenyl]$ | H | 1 | H |
| $-CH_2-CH_2-O-[4-Br-Phenyl]$ | H | 1 | H |
| $-CH_2-CH_2-O-[3-F-Phenyl]$ | H | 1 | H |
| $-CH_2-CH_2-O-[3-CH_3-Phenyl]$ | H | 1 | H |
| $-CH_2-CH_2-O-[4-CH_3-Phenyl]$ | H | 1 | H |
| $-CH_2-CH_2-O-[2,4,6-Cl_3-Phenyl]$ | H | 1 | H |
| $-CH_2-CH_2-[4-F-Phenyl]$ | H | 1 | H |
| $-CH_2-CH(CH_3)-CH_2-[4-F-Phenyl]$ | H | 1 | H |
| $-CH_2-CH_2-C(CH_3)_2-[4-Cl-Phenyl]$ | H | 1 | H |
| $-CH_2-CH_2-CH(CH_3)_2$ | H | 1 | H |
| $-CH_2-(CH_2)_3-O-[2-F-Phenyl]$ | H | 1 | H |
| $-CH_2-CH_2-$ 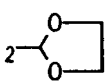 | H | 1 | H |
| $-CH_2$ 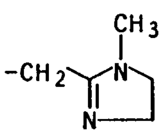 | H | 1 | H |
| $-CH_2-CH_2-N(C_2H_5)_2$ | H | 1 | H |
| $-CH_2-CH_2-$ 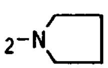 | H | 1 | H |

Tabelle (Forts.)

| $R^1$ | X | n | $R^2$ |
|---|---|---|---|
| $-CH_2-CH=CCl_2$ | H | 1 | H |
| $-CH_2-C(O)OCH_3$ | H | 1 | H |
| $-CH_2-CH_2-$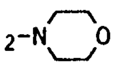 | H | 1 | H |
| $-CH_2-C(O)OCH_2-$Phenyl | H | 1 | H |
| $-CH_2-C(O)OCH_2-CH_3$ | H | 1 | H |
| $-CH_2C(O)-$Phenyl | H | 1 | H |
| $-CH_2-C(O)-CH_2-CH_3$ | H | 1 | H |
| $-CH_2-CH_2-CH_2-O-C(O)CH_3$ | H | 1 | H |
| $-CH_2-CH_2-CH_2-O-C(O)$Phenyl | H | 1 | H |
| $-C(O)-CH_3$ | H | 1 | H |
| $-CH_2-$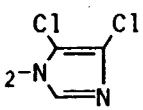 | H | 1 | H |
| $-CH_2-$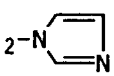 | H | 1 | H |
| $-CH_2-$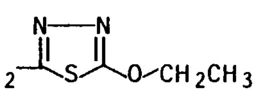 | H | 1 | H |
| $-C(O)-$Phenyl | H | 1 | H |

Tabelle (Forts.)

| $R^1$ | X | n | $R^2$ |
|---|---|---|---|
| 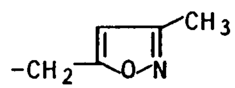 | H | 1 | H |
| 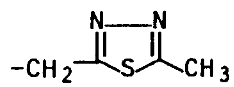 | H | 1 | H |
| 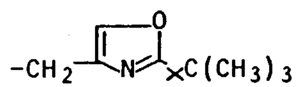 | H | 1 | H |
| 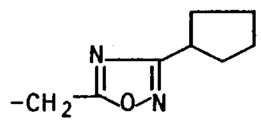 | H | 1 | H |
| 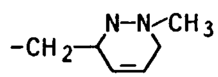 | H | 1 | H |
| 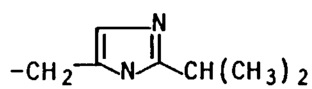 | H | 1 | H |
| 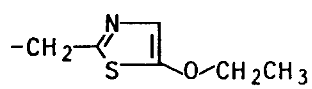 | H | 1 | H |

Tabelle (Forts.)

| $R^1$ | X | n | $R^2$ |
|---|---|---|---|
| 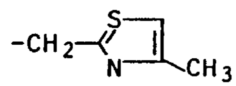 | H | 1 | H |
| 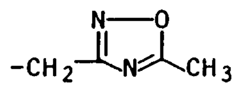 | H | 1 | H |
| 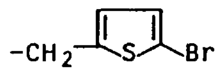 | H | 1 | H |
| 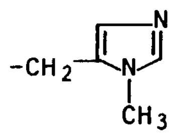 | H | 1 | H |
| 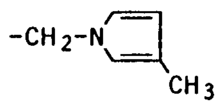 | H | 1 | H |
| 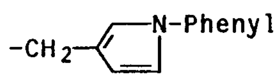 | H | 1 | H |
| 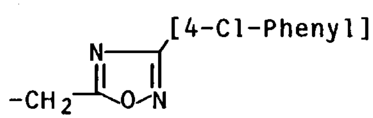 | H | 1 | H |

Tabelle (Forts.)

| $R^1$ | X | n | $R^2$ |
|---|---|---|---|
| 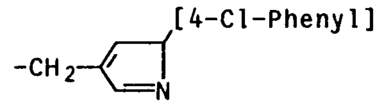 | H | 1 | H |
| 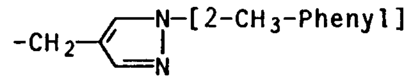 | H | 1 | H |
| 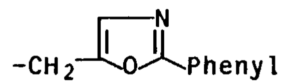 | H | 1 | H |
| 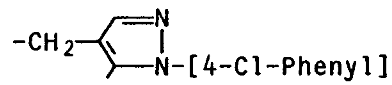 | H | 1 | H |
| 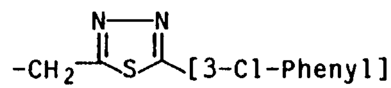 | H | 1 | H |
| 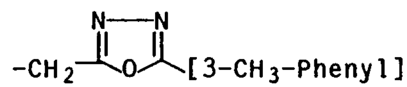 | H | 1 | H |
| $-CH_2-CH_2-S-$Phenyl | H | 1 | H |
| 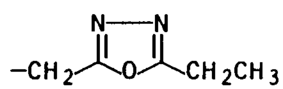 | H | 1 | H |

Tabelle (Forts.)

| $R^1$ | X | n | $R^2$ |
|---|---|---|---|
| 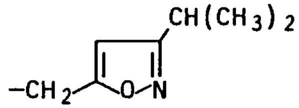 | H | 1 | H |
| 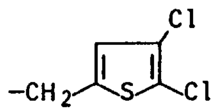 | H | 1 | H |
| 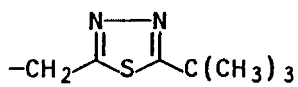 | H | 1 | H |
| 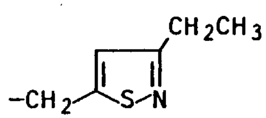 | H | 1 | H |
| 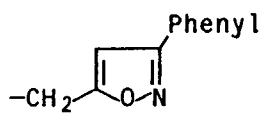 | H | 1 | H |

Tabelle (Forts.)

| $R^1$ | X | n | $R^2$ |
|---|---|---|---|
| 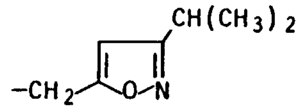 | H | 1 | H |
| 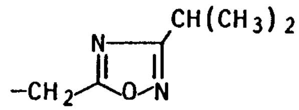 | H | 1 | H |
| 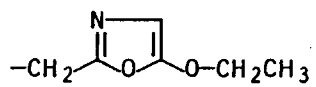 | H | 1 | H |
| 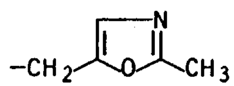 | H | 1 | H |
| 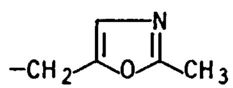 | H | 1 | H |
| 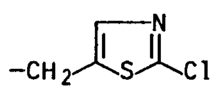 | H | 1 | H |
| 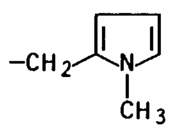 | H | 1 | H |

Tabelle (Forts.)

| $R^1$ | X | n | $R^2$ |
|---|---|---|---|
| 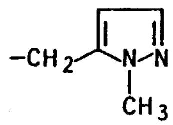 | H | 1 | H |
| 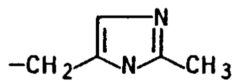 | H | 1 | H |
| 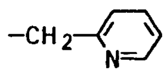 | H | 1 | H |
| 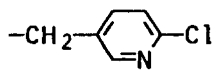 | H | 1 | H |
| 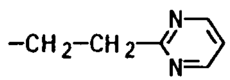 | H | 1 | H |
| 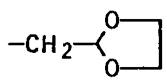 | H | 1 | H |
| 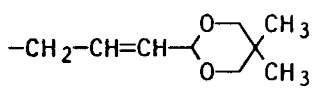 | H | 1 | H |

Tabelle (Forts.)

| $R^1$ | X | n | $R^2$ |
|---|---|---|---|
| 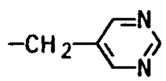 | H | 1 | H |
| 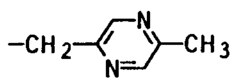 | H | 1 | H |
| 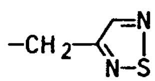 | H | 1 | H |
| 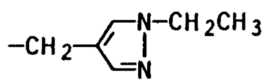 | H | 1 | H |
| 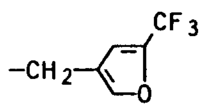 | H | 1 | H |
| 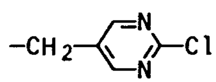 | H | 1 | H |
| 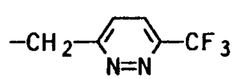 | H | 1 | H |

Tabelle (Forts.)

| $R^1$ | X | n | $R^2$ |
|---|---|---|---|
| 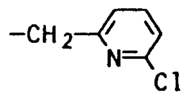 | H | 1 | H |
| 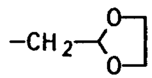 | H | 0 | $CH_3$ |
| 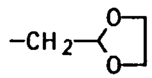 | H | 0 | $C_2H_5$ |
| 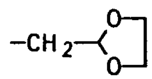 | H | 0 | $CH(CH_3)_2$ |
| 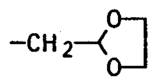 | $-CH_3$ | 0 | H |
| 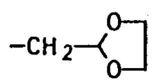 | $-CF_3$ | 0 | H |
| 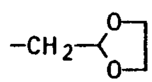 | -Cl | 0 | H |

Tabelle (Forts.)

| $R^1$ | X | n | $R^2$ |
|---|---|---|---|
| 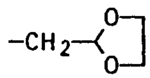 | $-COCH_3$ | 0 | H |
| 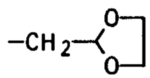 | $-COCCl_3$ | 0 | H |
| 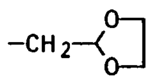 | $-CH(OH)CCl_3$ | 0 | H |
| 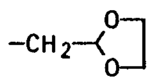 | $-COOC_2H_5$ | 0 | H |
| 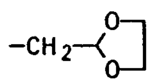 | -COO-Phenyl | 0 | H |
| 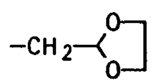 | -S-Phenyl | 0 | H |
| 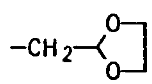 | $-CH_2$-S-Phenyl | 0 | H |

Tabelle (Forts.)

| $R^1$ | X | n | $R^2$ |
|---|---|---|---|
| $-CH_2-$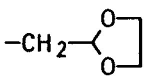 | -CO-Phenyl | 0 | H |
| $-CH_2-$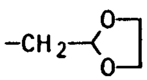 | -Phenyl | 0 | H |
| $-CH_2-$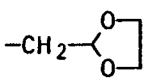 | $-S-(CH_2)_3CH_3$ | 0 | H |
| $CH_3$ | H | 0 | $CH_3$ |
| $CH_3$ | H | 0 | $C_2H_5$ |
| $CH_3$ | H | 0 | $CH_2(Ch_2)_2CH_3$ |
| $CH_3$ | $COCH_3$ | 0 | H |
| $CH_3$ | $CH_3$ | 0 | H |
| $CH_3$ | $CF_3$ | 0 | H |
| $CH_3$ | Cl | 0 | H |
| $CH_3$ | $CHF_2$ | 0 | H |
| $CH_3$ | $CH(OH)CCl_3$ | 0 | H |
| $CH_3$ | Br | 0 | H |
| $CH_3$ | $-CH_2-S-Phenyl$ | 0 | H |
| $CH_3$ | -S-Phenyl | 0 | H |
| $CH_3$ | -S-[4-Cl-Phenyl] | 0 | H |

Tabelle (Forts.)

| $R^1$ | X | n | $R^2$ |
|---|---|---|---|
| $CH_3$ | -Phenyl | 0 | H |
| $CH_3$ | $-COOC_2H_5$ | 0 | H |
| $CH_3$ | $-COOCH_3$ | 0 | H |
| $CH_3$ | -COO-Phenyl | 0 | H |
| $CH_3$ | -CO-Phenyl | 0 | H |
| $CH_3$ | $-COCCl_3$ | 0 | H |
| $-CH_2CH_3$ | H | 0 | H |
| $-CH_2CH_3$ | H | 0 | $CH_3$ |
| $-CH_2CH_3$ | $CH_2OCH_3$ | 0 | H |
| $-C(CH_3)_3$ | H | 0 | H |
| $-CH(CH_3)_2$ | $CH_3$ | 0 | H |
| $-CH(CH_3)_2$ | Cl | 0 | H |
| $-CH(CH_3)_2$ | $CH_2OH$ | 0 | H |
| $-CH(CH_3)_2$ | $CH(OH)CCl_3$ | 0 | H |
| $-CH(CH_3)_2$ | $CF_3$ | 0 | H |
| $-CH(CH_3)_2$ | H | 0 | $CH_3$ |
| $-CH_2C=CH_2$ | H | 0 | $CH_3$ |
| $-CH_2C\equiv CH$ | -COO-Phenyl | 0 | H |
| $-CH_2C\equiv CH$ | $-CH(OH)CCl_3$ | 0 | H |
| $-CH_2C\equiv CH$ | $-CH_3$ | 0 | H |
| $-CH_2CF_3$ | H | 0 | $CH_3$ |
| $-CH_2CF_3$ | H | 0 | $C_2H_5$ |
| $-CH_2CF_3$ | H | 0 | $CH(CH_3)_2$ |
| $-CH_2CF_3$ | $-CH(OH)CCl_3$ | 0 | H |

Tabelle (Forts.)

| $R^1$ | X | n | $R^2$ |
|---|---|---|---|
| $-CH_2CF_3$ | Cl | 0 | H |
| $-CH_2CF_3$ | $CF_3$ | 0 | H |
| $-CH_2CF_3$ | $CH(OH)CCl_3$ | 0 | H |
| $-CH_2CH_2CF{=}CF_2$ | H | 0 | H |
| $-CH_2CH_2CF{=}CF_2$ | H | 0 | $CH_3$ |
| $-CH_2CH_2CF{=}CF_2$ | Cl | 0 | H |
| $-CH_2CH_2CF{=}CF_2$ | $CH(OH)CCl_3$ | 0 | H |
| $-CH_2CH_2CF{=}CF_2$ | S-Phenyl | 0 | H |
| $-CH_2CH_2CF{=}CF_2$ | $-COOCH_3$ | 0 | H |
| $-CH_2$-Cyclopropyl | H | 0 | H |
| $-CH_2$-Cyclopropyl | H | 0 | $CH_3$ |
| $-CH_2$-Cyclopropyl | H | 0 | $CH(CH_3)_2$ |
| $-CH_2$-Cyclopropyl | $CF_3$ | 0 | H |
| $-CH_2$-Cyclopropyl | Cl | 0 | H |
| $-CH_2$-Cyclopropyl | $CH(OH)CCl_3$ | 0 | H |
| $-CH_2$-Cyclopropyl | -COO-Phenyl | 0 | H |
| $-CH_2$-Cyclopropyl | -CO-Phenyl | 0 | H |
| $-CH_2$-Cyclopropyl | $-COOC_2H_5$ | 0 | H |
| $-CH_2$-Cyclopropyl | H | 0 | H |
| $-CH_2$-Cyclopropyl | H | 0 | $CH_3$ |
| $-CH_2$-Cyclopropyl | H | 0 | $C_2H_5$ |
| $-CH_2$-Cyclopropyl | -S-Phenyl | 0 | H |
| $-CH_2$-Cyclopropyl | $-S-(CH_2)_3CH_3$ | 0 | H |
| $-CH_2$-Cyclopropyl | $-COCH_3$ | 0 | H |

Tabelle (Forts.)

| $R^1$ | X | n | $R^2$ |
|---|---|---|---|
| $-CH_2$-Cyclopropyl | $-COCCl_3$ | 0 | H |
| $-CH_2$-Cyclopropyl | $-CH_2$-S-Phenyl | 0 | H |
| $-CH_2$-Cyclopropyl | $-CH_3$ | 0 | H |
| $-CH_2CH_2OCH_3$ | H | 0 | H |
| $-CH_2CH_2OCH_3$ | H | 0 | $CH_3$ |
| $-CH_2CH_2OCH_3$ | H | 0 | $C_2H_5$ |
| $-CH_2CH_2OCH_3$ | H | 0 | $CH(CH_3)_2$ |
| $-CH_2CH_2OCH_3$ | Cl | 0 | H |
| $-CH_2CH_2OCH_3$ | $CH_3$ | 0 | H |
| $-CH_2CH_2OCH_3$ | $COCH_3$ | 0 | H |
| $-CH_2CH_2OCH_3$ | $C(OH)CCl_3$ | 0 | H |
| $-CH_2CH_2OCH_3$ | $COCF_3$ | 0 | H |
| $-CH_2CH_2OCH_3$ | -S-Phenyl | 0 | H |
| $-CH_2CH_2OCH_3$ | $-CH_2$-S-Phenyl | 0 | H |
| $-CH_2CH_2OCH_3$ | $-CHF_2$ | 0 | H |
| $-CH_2CH_2OCH_3$ | $-COOCH_3$ | 0 | H |
| $-CH_2CH_2OCH_3$ | -COO-Phenyl | 0 | H |
| $-CH_2CH_2CH_2N(C_2H_5)_2$ | H | 0 | H |
| $-CH_2CH_2CH_2N(C_2H_5)_2$ | $-CH_3$ | 0 | H |
| $-CH_2CH_2CH_2N(C_2H_5)_2$ | $-CHF_2$ | 0 | H |
| $-CH_2CH_2CH_2N(C_2H_5)_2$ | H | 0 | $-CH_3$ |
| $-CH_2CH_2CH_2N(C_2H_5)_2$ | H | 0 | $-C_2H_5$ |
| $-CH_2CH_2CH_2N(C_2H_5)_2$ | H | 0 | $-CH_2-(CH_2)_2-CH_3$ |
| $-CH_2CH_2CH_2N(C_2H_5)_2$ | -Cl | 0 | H |

Tabelle (Forts.)

| $R^1$ | X | n | $R^2$ |
|---|---|---|---|
| $-CH_2-CH_2-CH_2-N(C_2H_5)_2$ | $-COCH_3$ | 0 | H |
| $-CH_2-CH_2-CH_2-N(C_2H_5)_2$ | $-COCF_3$ | 0 | H |
| $-CH_2-CH_2-CH_2-N(C_2H_5)_2$ | $-C(OH)CCl_3$ | 0 | H |
| $-CH_2-CH_2-CH_2-N(C_2H_5)_2$ | $-COOC_2H_5$ | 0 | H |
| $-CH_2-CH_2-CH_2-N(C_2H_5)_2$ | -COO-Phenyl | 0 | H |
| $-CH_2-CH_2-CH_2-N(C_2H_5)_2$ | $-CH_2$-S-Phenyl | 0 | H |
| $-CH_2-CH_2-OCH_2-CF_3$ | H | 0 | H |
| $-CH_2-CH_2-OCH_2-CF_3$ | H | 0 | $-CH_3$ |
| $-CH_2-CH_2-OCH_2-CF_3$ | H | 0 | $-C_2H_5$ |
| $-CH_2-CH_2-OCH_2-CF_3$ | H | 0 | $-CH(CH_3)_2$ |
| $-CH_2-CH_2-OCH_2-CF_3$ | -Br | 0 | H |
| $-CH_2-CH_2-OCH_2-CF_3$ | $-CF_3$ | 0 | H |
| $-CH_2-CH_2-OCH_2-CF_3$ | $-CH_3$ | 0 | H |
| $-CH_2-CH_2-OCH_2-CF_3$ | $-COCH_3$ | 0 | H |
| $-CH_2-CH_2-OCH_2-CF_3$ | $-COCCl_3$ | 0 | H |
| $-CH_2-CH_2-OCH_2-CF_3$ | $-COOC_2H_5$ | 0 | H |
| $-CH_2-CH_2-OCH_2-CF_3$ | -COO-Phenyl | 0 | H |
| $-CH_2-CH_2-OCH_2-CF_3$ | $-CH_2$-S-Phenyl | 0 | H |
| $-CH_2-CH_2-OCH_2-CF_3$ | -S-Phenyl | 0 | H |
| $-CH_2-CH_2-S-CH_3$ | H | 0 | $CH_3$ |
| $-CH_2-CH_2-S-CH_3$ | H | 0 | $C_2H_5$ |
| $-CH_2-CH_2-S-CH_3$ | H | 0 | $-CH(CH_3)_2$ |
| $-CH_2-CH_2-S-CH_3$ | -Cl | 0 | H |
| $-CH_2-CH_2-S-CH_3$ | $-CHF_2$ | 0 | H |

Tabelle (Forts.)

| $R^1$ | X | n | $R^2$ |
|---|---|---|---|
| $-CH_2-CH_2-S-CH_3$ | $-CH(OH)CCl_3$ | 0 | H |
| $-CH_2-CH_2-S-CH_3$ | $-CH_3$ | 0 | H |
| $-CH_2-CH_2-S-CH_3$ | $-COCH_3$ | 0 | H |
| $-CH_2-CH_2-S-CH_3$ | $-COCF_3$ | 0 | H |
| $-CH_2-CH_2-S-CH_3$ | $-COOCH_3$ | 0 | H |
| $-CH_2-CH_2-S-CH_3$ | -COO-Phenyl | 0 | H |
| $-CH_2-CH_2-S-CH_3$ | -CO-Phenyl | 0 | H |
| $-CH_2-CH_2-S-CH_3$ | -S-Phenyl | 0 | H |
| $-CH_2-CH_2-S-CH_3$ | $-CH_2$-S-Phenyl | 0 | H |
| $-CH_2-CH_2-SCHF_2$ | H | 0 | $CH_3$ |
| $-CH_2-CH_2-SCHF_2$ | H | 0 | $C_2H_5$ |
| $-CH_2-CH_2-SCHF_2$ | H | 0 | $CH(CH_3)_2$ |
| $-CH_2-CH_2-SCHF_2$ | $-CH_3$ | 0 | H |
| $-CH_2-CH_2-SCHF_2$ | $-CF_3$ | 0 | H |
| $-CH_2-CH_2-SCHF_2$ | -Cl | 0 | H |
| $-CH_2-CH_2-SCHF_2$ | $-COCH_3$ | 0 | H |
| $-CH_2-CH_2-SCHF_2$ | $-COCCl_3$ | 0 | H |
| $-CH_2-CH_2-SCHF_2$ | $-CH(OH)CCl_3$ | 0 | H |
| $-CH_2-CH_2-SCHF_2$ | $-COOC_2H_5$ | 0 | H |
| $-CH_2-CH_2-SCHF_2$ | -COO-Phenyl | 0 | H |
| $-CH_2-CH_2-SCHF_2$ | -S-Phenyl | 0 | H |
| $-CH_2-CH_2-SCHF_2$ | $-CH_2$-S-Phenyl | 0 | H |
| $-CH_2-CH_2-SCHF_2$ | -CO-Phenyl | 0 | H |
| $-CH_2-CH_2-SCHF_2$ | -Phenyl | 0 | H |

Tabelle (Forts.)

| $R^1$ | X | n | $R^2$ |
|---|---|---|---|
| $-CH_2-CH_2-SCHF_2$ | $-S-(CH_2)_3CH_3$ | 0 | H |
| $-CH_2$-Phenyl | H | 0 | $CH_3$ |
| $-CH_2$-Phenyl | H | 0 | $C_2H_5$ |
| $-CH_2$-Phenyl | H | 0 | $CH(CH_3)_2$ |
| $-CH_2$-Phenyl | $-CH_3$ | 0 | H |
| $-CH_2$-Phenyl | $-CF_3$ | 0 | H |
| $-CH_2$-Phenyl | -Cl | 0 | H |
| $-CH_2$-Phenyl | $-COCH_3$ | 0 | H |
| $-CH_2$-Phenyl | $-COCCl_3$ | 0 | H |
| $-CH_2$-Phenyl | $-CH(OH)CCl_3$ | 0 | H |
| $-CH_2$-Phenyl | $-COOC_2H_5$ | 0 | H |
| $-CH_2$-Phenyl | -COO-Phenyl | 0 | H |
| $-CH_2$-Phenyl | -S-Phenyl | 0 | H |
| $-CH_2$-Phenyl | $-CH_2$-S-Phenyl | 0 | H |
| $-CH_2$-Phenyl | -CO-Phenyl | 0 | H |
| $-CH_2$-Phenyl | -Phenyl | 0 | H |
| $-CH_2$-Phenyl | $-S-(CH_2)_3CH_3$ | 0 | H |
| $-CH_2$-[2,4-$Cl_2$-Phenyl] | H | 0 | $CH_3$ |
| $-CH_2$-[2,4-$Cl_2$-Phenyl] | H | 0 | $C_2H_5$ |
| $-CH_2$-[2,4-$Cl_2$-Phenyl] | H | 0 | $CH(CH_3)_2$ |
| $-CH_2$-[2,4-$Cl_2$-Phenyl] | $-CH_3$ | 0 | H |
| $-CH_2$-[2,4-$Cl_2$-Phenyl] | $-CF_3$ | 0 | H |
| $-CH_2$-[2,4-$Cl_2$-Phenyl] | -Cl | 0 | H |
| $-CH_2$-[2,4-$Cl_2$-Phenyl] | $-COCH_3$ | 0 | H |

Tabelle (Forts.)

| $R^1$ | X | n | $R^2$ |
|---|---|---|---|
| $-CH_2-[2,4-Cl_2-Phenyl]$ | $-COCCl_3$ | 0 | H |
| $-CH_2-[2,4-Cl_2-Phenyl]$ | $-CH(OH)CCl_3$ | 0 | H |
| $-CH_2-[2,4-Cl_2-Phenyl]$ | $-COOC_2H_5$ | 0 | H |
| $-CH_2-[2,4-Cl_2-Phenyl]$ | -COO-Phenyl | 0 | H |
| $-CH_2-[2,4-Cl_2-Phenyl]$ | -S-Phenyl | 0 | H |
| $-CH_2-[2,4-Cl_2-Phenyl]$ | $-CH_2-S-Phenyl$ | 0 | H |
| $-CH_2-[2,4-Cl_2-Phenyl]$ | -CO-Phenyl | 0 | H |
| $-CH_2-[2,4-Cl_2-Phenyl]$ | -Phenyl | 0 | H |
| $-CH_2-[2,4-Cl_2-Phenyl]$ | $-S-(CH_2)_3CH_3$ | 0 | H |
| $-CH_2-CH=CH-CH_2-[4-F-Phenyl]$ | H | 0 | $CH_3$ |
| $-CH_2-CH=CH-CH_2-[4-F-Phenyl]$ | H | 0 | $C_2H_5$ |
| $-CH_2-CH=CH-CH_2-[4-F-Phenyl]$ | H | 0 | $CH(CH_3)_2$ |
| $-CH_2-CH=CH-CH_2-[4-F-Phenyl]$ | $-CH_3$ | 0 | H |
| $-CH_2-CH=CH-CH_2-[4-F-Phenyl]$ | $-CF_3$ | 0 | H |
| $-CH_2-CH=CH-CH_2-[4-F-Phenyl]$ | -Cl | 0 | H |
| $-CH_2-CH=CH-CH_2-[4-F-Phenyl]$ | $-COCH_3$ | 0 | H |
| $-CH_2-CH=CH-CH_2-[4-F-Phenyl]$ | $-COCCl_3$ | 0 | H |
| $-CH_2-CH=CH-CH_2-[4-F-Phenyl]$ | $-CH(OH)CCl_3$ | 0 | H |
| $-CH_2-CH=CH-CH_2-[4-F-Phenyl]$ | $-COOC_2H_5$ | 0 | H |
| $-CH_2-CH=CH-CH_2-[4-F-Phenyl]$ | -COO-Phenyl | 0 | H |
| $-CH_2-CH=CH-CH_2-[4-F-Phenyl]$ | -S-Phenyl | 0 | H |
| $-CH_2-CH=CH-CH_2-[4-F-Phenyl]$ | $-CH_2-S-Phenyl$ | 0 | H |
| $-CH_2-CH=CH-CH_2-[4-F-Phenyl]$ | -CO-Phenyl | 0 | H |
| $-CH_2-CH=CH-CH_2-[4-F-Phenyl]$ | -Phenyl | 0 | H |

Tabelle (Forts.)

| $R^1$ | X | n | $R^2$ |
|---|---|---|---|
| $-CH_2-CH=CH-CH_2-$[4-F-Phenyl] | $-S-(CH_2)_3CH_3$ | 0 | H |
| $-CH_2-(CH_2)_2-O-$Phenyl | H | 0 | $CH_3$ |
| $-CH_2-(CH_2)_2-O-$Phenyl | H | 0 | $C_2H_5$ |
| $-CH_2-(CH_2)_2-O-$Phenyl | H | 0 | $CH(CH_3)_2$ |
| $-CH_2-(CH_2)_2-O-$Phenyl | $-CH_3$ | 0 | H |
| $-CH_2-(CH_2)_2-O-$Phenyl | $-CF_3$ | 0 | H |
| $-CH_2-(CH_2)_2-O-$Phenyl | -Cl | 0 | H |
| $-CH_2-(CH_2)_2-O-$Phenyl | $-COCH_3$ | 0 | H |
| $-CH_2-(CH_2)_2-O-$Phenyl | $-COCCl_3$ | 0 | H |
| $-CH_2-(CH_2)_2-O-$Phenyl | $-CH(OH)CCl_3$ | 0 | H |
| $-CH_2-(CH_2)_2-O-$Phenyl | $-COOC_2H_5$ | 0 | H |
| $-CH_2-(CH_2)_2-O-$Phenyl | -COO-Phenyl | 0 | H |
| $-CH_2-(CH_2)_2-O-$Phenyl | -S-Phenyl | 0 | H |
| $-CH_2-(CH_2)_2-O-$Phenyl | $-CH_2-S-$Phenyl | 0 | H |
| $-CH_2-(CH_2)_2-O-$Phenyl | -CO-Phenyl | 0 | H |
| $-CH_2-(CH_2)_2-O-$Phenyl | -Phenyl | 0 | H |
| $-CH_2-(CH_2)_2-O-$Phenyl | $-S-(CH_2)_3CH_3$ | 0 | H |
| 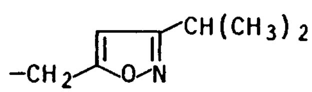 | H | 0 | $CH_3$ |
| 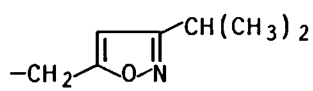 | H | 0 | $C_2H_5$ |

Tabelle (Forts.)

| $R^1$ | X | n | $R^2$ |
|---|---|---|---|
| 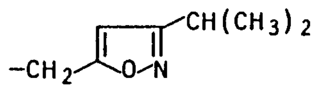 | H | 0 | $CH(CH_3)_2$ |
| 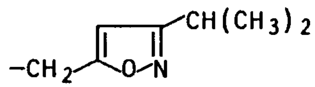 | $-CH_3$ | 0 | H |
| 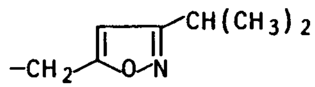 | $-CF_3$ | 0 | H |
| 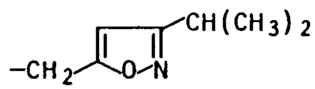 | -Cl | 0 | H |
| 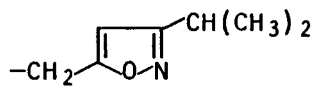 | $-COCH_3$ | 0 | H |
| 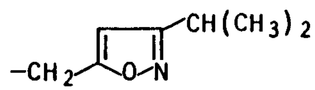 | $-COCCl_3$ | 0 | H |

Tabelle (Forts.)

| $R^1$ | X | n | $R^2$ |
|---|---|---|---|
| 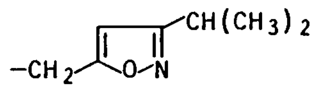 | $-CH(OH)CCl_3$ | 0 | H |
| 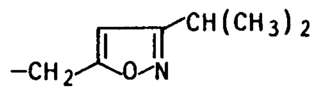 | $-COOC_2H_5$ | 0 | H |
| 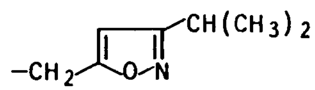 | -COO-Phenyl | 0 | H |
| 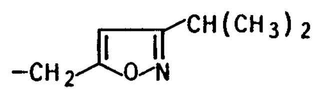 | -S-Phenyl | 0 | H |
| 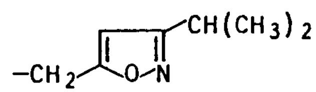 | $-CH_2$-S-Phenyl | 0 | H |
| 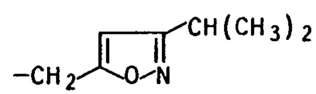 | -CO-Phenyl | 0 | H |

Tabelle (Forts.)

| $R^1$ | X | n | $R^2$ |
|---|---|---|---|
| 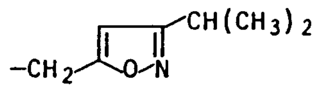 | -Phenyl | 0 | H |
| 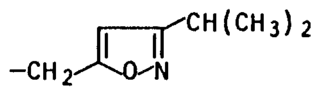 | $-S-(CH_2)_3CH_3$ | 0 | H |
| 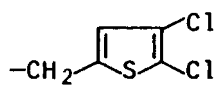 | H | 0 | $CH_3$ |
| 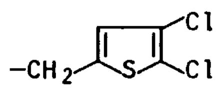 | H | 0 | $C_2H_5$ |
| 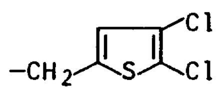 | H | 0 | $CH(CH_3)_2$ |
| 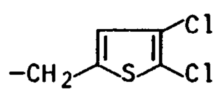 | $-CH_3$ | 0 | H |
| 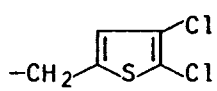 | $-CF_3$ | 0 | H |

Tabelle (Forts.)

| $R^1$ | X | n | $R^2$ |
|---|---|---|---|
| 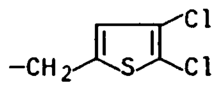 | -Cl | 0 | H |
| 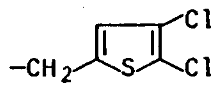 | $-COCH_3$ | 0 | H |
| 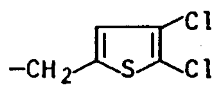 | $-COCCl_3$ | 0 | H |
| 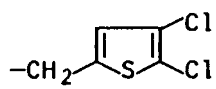 | $-CH(OH)CCl_3$ | 0 | H |
| 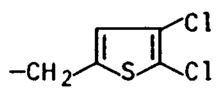 | $-COOC_2H_5$ | 0 | H |
| 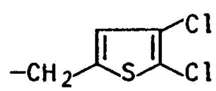 | -COO-Phenyl | 0 | H |
| 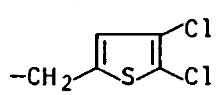 | -S-Phenyl | 0 | H |

Tabelle (Forts.)

| $R^1$ | X | n | $R^2$ |
|---|---|---|---|
| 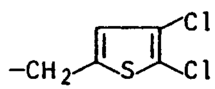 | $-CH_2$-S-Phenyl | 0 | H |
| 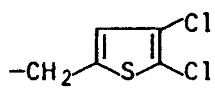 | -CO-Phenyl | 0 | H |
| 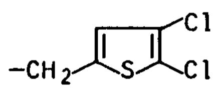 | -Phenyl | 0 | H |
| 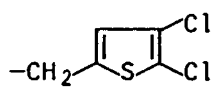 | $-S-(CH_2)_3CH_3$ | 0 | H |
| 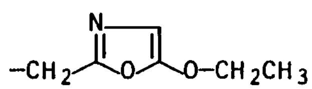 | H | 0 | $CH_3$ |
| 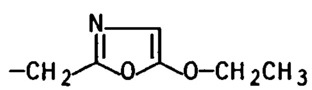 | H | 0 | $C_2H_5$ |
| 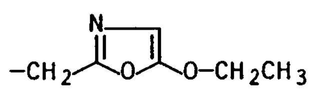 | H | 0 | $CH(CH_3)_2$ |

Tabelle (Forts.)

| $R^1$ | X | n | $R^2$ |
|---|---|---|---|
| 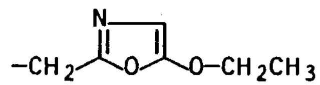 | $-CH_3$ | 0 | H |
| 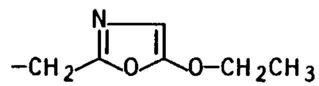 | $-CF_3$ | 0 | H |
| 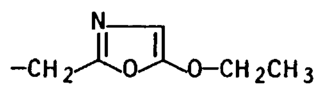 | $-Cl$ | 0 | H |
| 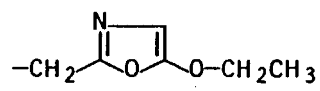 | $-COCH_3$ | 0 | H |
| 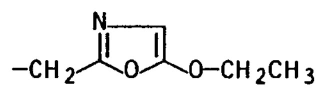 | $-COCCl_3$ | 0 | H |
| 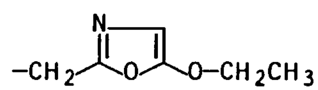 | $-CH(OH)CCl_3$ | 0 | H |
| 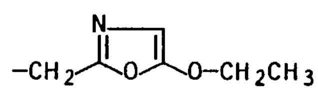 | $-COOC_2H_5$ | 0 | H |

Tabelle (Forts.)

| $R^1$ | X | n | $R^2$ |
|---|---|---|---|
| 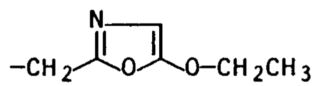 | -COO-Phenyl | 0 | H |
| 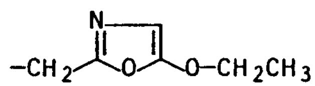 | -S-Phenyl | 0 | H |
| 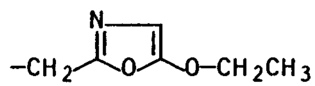 | $-CH_2-S-$Phenyl | 0 | H |
| 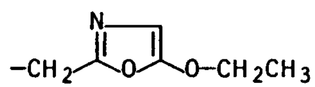 | -CO-Phenyl | 0 | H |
| 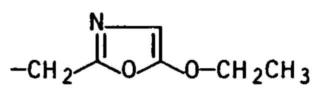 | -Phenyl | 0 | H |
| 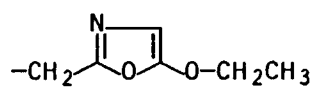 | $-S-(CH_2)_3CH_3$ | 0 | H |
| 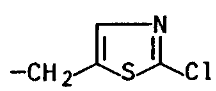 | H | 0 | $CH_3$ |

Tabelle (Forts.)

| $R^1$ | X | n | $R^2$ |
|---|---|---|---|
| 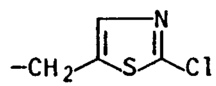 | H | 0 | $C_2H_5$ |
| 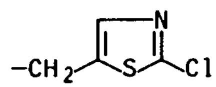 | H | 0 | $CH(CH_3)_2$ |
| 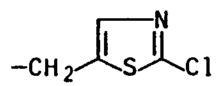 | $-CH_3$ | 0 | H |
| 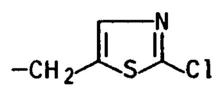 | $-CF_3$ | 0 | H |
| 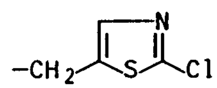 | -Cl | 0 | H |
| 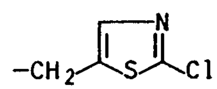 | $-COCH_3$ | 0 | H |
| 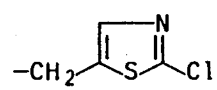 | $-COCCl_3$ | 0 | H |

Tabelle (Forts.)

| $R^1$ | X | n | $R^2$ |
|---|---|---|---|
| 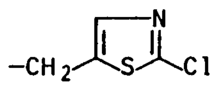 | $-CH(OH)CCl_3$ | 0 | H |
| 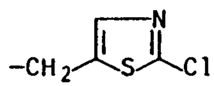 | $-COOC_2H_5$ | 0 | H |
| 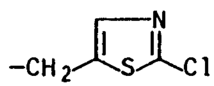 | -COO-Phenyl | 0 | H |
| 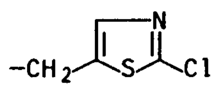 | -S-Phenyl | 0 | H |
| 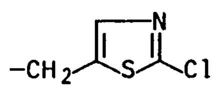 | $-CH_2$-S-Phenyl | 0 | H |
| 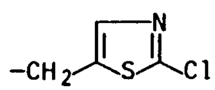 | -CO-Phenyl | 0 | H |
| 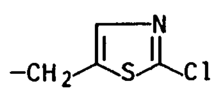 | -Phenyl | 0 | H |

Tabelle (Forts.)

| $R^1$ | X | n | $R^2$ |
|---|---|---|---|
| 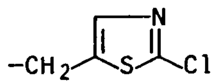 | $-S-(CH_2)_3CH_3$ | 0 | H |
| 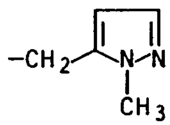 | H | 0 | $CH_3$ |
| 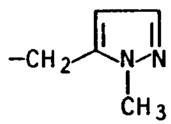 | H | 0 | $C_2H_5$ |
| 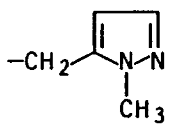 | H | 0 | $CH(CH_3)_2$ |
| 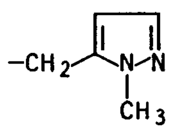 | $-CH_3$ | 0 | H |
| 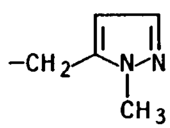 | $-CF_3$ | 0 | H |

Tabelle (Forts.)

| $R^1$ | X | n | $R^2$ |
|---|---|---|---|
| 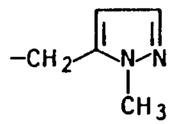 | $-Cl$ | 0 | H |
| 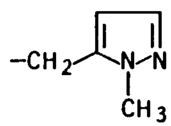 | $-COCH_3$ | 0 | H |
| 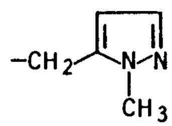 | $-COCCl_3$ | 0 | H |
| 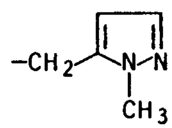 | $-CH(OH)CCl_3$ | 0 | H |
| 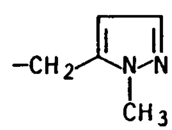 | $-COOC_2H_5$ | 0 | H |
| 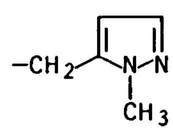 | -COO-Phenyl | 0 | H |

Tabelle (Forts.)

| $R^1$ | X | n | $R^2$ |
|---|---|---|---|
| 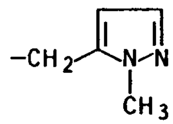 | -S-Phenyl | 0 | H |
| 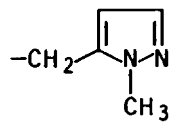 | $-CH_2$-S-Phenyl | 0 | H |
| 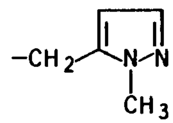 | -CO-Phenyl | 0 | H |
| 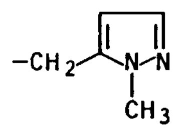 | -Phenyl | 0 | H |
| 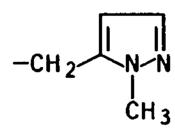 | $-S-(CH_2)_3CH_3$ | 0 | H |
| 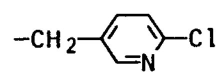 | H | 0 | $CH_3$ |

EP 0 508 190 B1

Tabelle (Forts.)

| $R^1$ | X | n | $R^2$ |
|---|---|---|---|
| 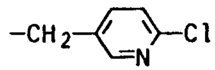 | H | 0 | $C_2H_5$ |
| 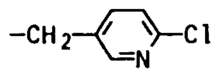 | H | 0 | $CH(CH_3)_2$ |
| 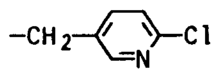 | $-CH_3$ | 0 | H |
| 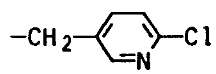 | $-CF_3$ | 0 | H |
| 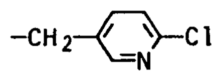 | $-Cl$ | 0 | H |
| 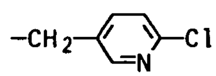 | $-COCH_3$ | 0 | H |
| 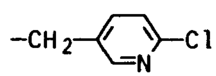 | $-COCCl_3$ | 0 | H |

50

Tabelle (Forts.)

| $R^1$ | X | n | $R^2$ |
|---|---|---|---|
| 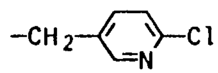 | $-CH(OH)CCl_3$ | 0 | H |
| 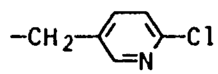 | $-COOC_2H_5$ | 0 | H |
| 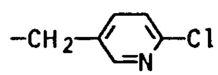 | -COO-Phenyl | 0 | H |
| 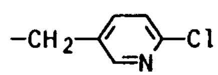 | -S-Phenyl | 0 | H |
| 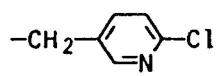 | $-CH_2$-S-Phenyl | 0 | H |
| 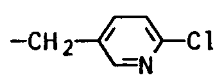 | -CO-Phenyl | 0 | H |
| 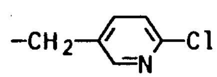 | -Phenyl | 0 | H |

Tabelle (Forts.)

| $R^1$ | X | n | $R^2$ |
|---|---|---|---|
| 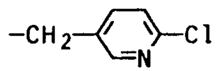 | $-S-(CH_2)_3CH_3$ | 0 | H |
| 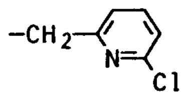 | H | 0 | $CH_3$ |
| 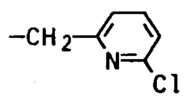 | H | 0 | $C_2H_5$ |
| 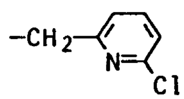 | H | 0 | $CH(CH_3)_2$ |
| 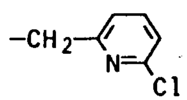 | $-CH_3$ | 0 | H |
| 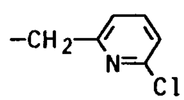 | $-CF_3$ | 0 | H |

Tabelle (Forts.)

| $R^1$ | X | n | $R^2$ |
|---|---|---|---|
| $-CH_2-$ 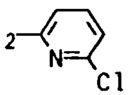 | $-Cl$ | 0 | H |
| $-CH_2-$ 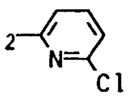 | $-COCH_3$ | 0 | H |
| $-CH_2-$ 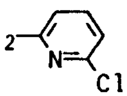 | $-COCCl_3$ | 0 | H |
| $-CH_2-$ 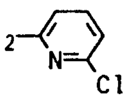 | $-CH(OH)CCl_3$ | 0 | H |
| $-CH_2-$ 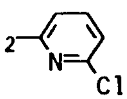 | $-COOC_2H_5$ | 0 | H |
| $-CH_2-$ 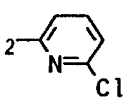 | -COO-Phenyl | 0 | H |

Tabelle (Forts.)

| $R^1$ | X | n | $R^2$ |
|---|---|---|---|
| $-CH_2-$ 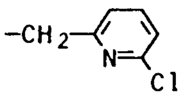 | -S-Phenyl | 0 | H |
| $-CH_2-$ 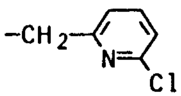 | $-CH_2-S-$Phenyl | 0 | H |
| $-CH_2-$ 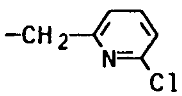 | -CO-Phenyl | 0 | H |
| $-CH_2-$ 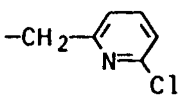 | -Phenyl | 0 | H |
| $-CH_2-$ 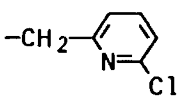 | $-S-(CH_2)_3CH_3$ | 0 | H |
| $-CH_2-$ 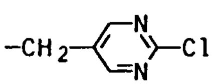 | H | 0 | $CH_3$ |

Tabelle (Forts.)

| $R^1$ | X | n | $R^2$ |
|---|---|---|---|
| 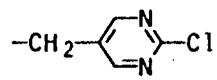 | H | 0 | $C_2H_5$ |
| 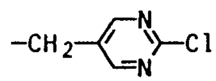 | H | 0 | $CH(CH_3)_2$ |
| 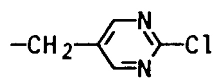 | $-CH_3$ | 0 | H |
| 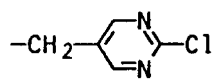 | $-CF_3$ | 0 | H |
| 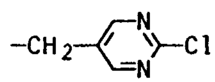 | -Cl | 0 | H |
| 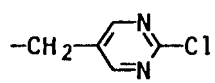 | $-COCH_3$ | 0 | H |
| 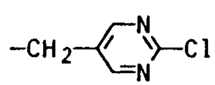 | $-COCl_3$ | 0 | H |

Tabelle (Forts.)

| $R^1$ | X | n | $R^2$ |
|---|---|---|---|
| 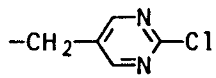 | $-CH(OH)CCl_3$ | 0 | H |
| 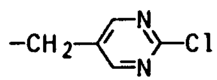 | $-COOC_2H_5$ | 0 | H |
| 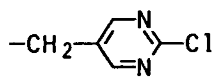 | -COO-Phenyl | 0 | H |
| 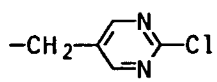 | -S-Phenyl | 0 | H |
| 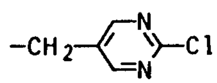 | $-CH_2$-S-Phenyl | 0 | H |
| 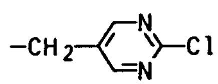 | -CO-Phenyl | 0 | H |
| 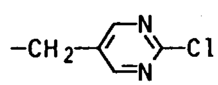 | -Phenyl | 0 | H |

Tabelle (Forts.)

| $R^1$ | X | n | $R^2$ |
|---|---|---|---|
| 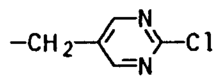 | $-S-(CH_2)_3CH_3$ | 0 | H |
| $-CH_3$ | H | 1 | $CH_3$ |
| $-CH_3$ | H | 1 | $C_2H_5$ |
| $-CH_3$ | H | 1 | $CH(CH_3)_2$ |
| $-CH_3$ | $-CH_3$ | 1 | H |
| $-CH_3$ | $-CF_3$ | 1 | H |
| $-CH_3$ | -Cl | 1 | H |
| $-CH_3$ | $-COCH_3$ | 1 | H |
| $-CH_3$ | $-COCCl_3$ | 1 | H |
| $-CH_3$ | $-CH(OH)CCl_3$ | 1 | H |
| $-CH_3$ | $-COOC_2H_5$ | 1 | H |
| $-CH_3$ | -COO-Phenyl | 1 | H |
| $-CH_3$ | -S-Phenyl | 1 | H |
| $-CH_3$ | $-CH_2-S-Phenyl$ | 1 | H |
| $-CH_3$ | -CO-Phenyl | 1 | H |
| $-CH_3$ | -Phenyl | 1 | H |
| $-CH_3$ | $-S-(CH_2)_3CH_3$ | 1 | H |
| $-C_2H_5$ | H | 1 | $CH_3$ |
| $-C_2H_5$ | H | 1 | $C_2H_5$ |

Tabelle (Forts.)

| $R^1$ | X | n | $R^2$ |
|---|---|---|---|
| $-C_2H_5$ | H | 1 | $CH(CH_3)_2$ |
| $-C_2H_5$ | $-CH_3$ | 1 | H |
| $-C_2H_5$ | $-CF_3$ | 1 | H |
| $-C_2H_5$ | -Cl | 1 | H |
| $-C_2H_5$ | $-COCH_3$ | 1 | H |
| $-C_2H_5$ | $-COCCl_3$ | 1 | H |
| $-C_2H_5$ | $-CH(OH)CCl_3$ | 1 | H |
| $-C_2H_5$ | $-COOC_2H_5$ | 1 | H |
| $-C_2H_5$ | -COO-Phenyl | 1 | H |
| $-C_2H_5$ | -S-Phenyl | 1 | H |
| $-C_2H_5$ | $-CH_2$-S-Phenyl | 1 | H |
| $-C_2H_5$ | -CO-Phenyl | 1 | H |
| $-C_2H_5$ | -Phenyl | 1 | H |
| $-C_2H_5$ | $-S-(CH_2)_3CH_3$ | 1 | H |
| $-CH(CH_3)_2$ | H | 1 | $CH_3$ |
| $-CH(CH_3)_2$ | H | 1 | $C_2H_5$ |
| $-CH(CH_3)_2$ | H | 1 | $CH(CH_3)_2$ |
| $-CH(CH_3)_2$ | $-CH_3$ | 1 | H |
| $-CH(CH_3)_2$ | $-CF_3$ | 1 | H |
| $-CH(CH_3)_2$ | -Cl | 1 | H |
| $-CH(CH_3)_2$ | $-COCH_3$ | 1 | H |
| $-CH(CH_3)_2$ | $-COCCl_3$ | 1 | H |
| $-CH(CH_3)_2$ | $-CH(OH)CCl_3$ | 1 | H |
| $-CH(CH_3)_2$ | $-COOC_2H_5$ | 1 | H |

Tabelle (Forts.)

| $R^1$ | X | n | $R^2$ |
|---|---|---|---|
| $-CH(CH_3)_2$ | -COO-Phenyl | 1 | H |
| $-CH(CH_3)_2$ | -S-Phenyl | 1 | H |
| $-CH(CH_3)_2$ | $-CH_2$-S-Phenyl | 1 | H |
| $-CH(CH_3)_2$ | -CO-Phenyl | 1 | H |
| $-CH(CH_3)_2$ | -Phenyl | 1 | H |
| $-CH(CH_3)_2$ | $-S-(CH_2)_3CH_3$ | 1 | H |
| $-CH_2-CH-(CH_3)_2$ | H | 1 | $CH_3$ |
| $-CH_2-CH-(CH_3)_2$ | H | 1 | $C_2H_5$ |
| $-CH_2-CH-(CH_3)_2$ | H | 1 | $CH(CH_3)_2$ |
| $-CH_2-CH-(CH_3)_2$ | $-CH_3$ | 1 | H |
| $-CH_2-CH-(CH_3)_2$ | $-CF_3$ | 1 | H |
| $-CH_2-CH-(CH_3)_2$ | -Cl | 1 | H |
| $-CH_2-CH-(CH_3)_2$ | $-COCH_3$ | 1 | H |
| $-CH_2-CH-(CH_3)_2$ | $-COCCl_3$ | 1 | H |
| $-CH_2-CH-(CH_3)_2$ | $-CH(OH)CCl_3$ | 1 | H |
| $-CH_2-CH-(CH_3)_2$ | $-COOC_2H_5$ | 1 | H |
| $-CH_2-CH-(CH_3)_2$ | -COO-Phenyl | 1 | H |
| $-CH_2-CH-(CH_3)_2$ | -S-Phenyl | 1 | H |
| $-CH_2-CH-(CH_3)_2$ | $-CH_2$-S-Phenyl | 1 | H |
| $-CH_2-CH-(CH_3)_2$ | -CO-Phenyl | 1 | H |
| $-CH_2-CH-(CH_3)_2$ | -Phenyl | 1 | H |
| $-CH_2-CH-(CH_3)_2$ | $-S-(CH_2)_3CH_3$ | 1 | H |
| $-CH_2-CH=CH_2$ | H | 1 | $CH(CH_3)_2$ |
| $-CH_2-CH=CH_2$ | $-CH_3$ | 1 | H |

Tabelle (Forts.)

| $R^1$ | X | n | $R^2$ |
|---|---|---|---|
| $-CH_2-CH=CH_2$ | $-CF_3$ | 1 | H |
| $-CH_2-CH=CH_2$ | $-Cl$ | 1 | H |
| $-CH_2-CH=CH_2$ | $-COCH_3$ | 1 | H |
| $-CH_2-CH=CH_2$ | $-COCCl_3$ | 1 | H |
| $-CH_2-CH=CH_2$ | $-CH(OH)CCl_3$ | 1 | H |
| $-CH_2-CH=CH_2$ | $-COOC_2H_5$ | 1 | H |
| $-CH_2-CH=CH_2$ | -COO-Phenyl | 1 | H |
| $-CH_2-CH=CH_2$ | -S-Phenyl | 1 | H |
| $-CH_2-CH=CH_2$ | $-CH_2$-S-Phenyl | 1 | H |
| $-CH_2-CH=CH_2$ | -CO-Phenyl | 1 | H |
| $-CH_2-CH=CH_2$ | -Phenyl | 1 | H |
| $-CH_2-CH=CH_2$ | $-S-(CH_2)_3CH_3$ | 1 | H |
| $-CH_2C\equiv CH$ | H | 1 | $CH_3$ |
| $-CH_2C\equiv CH$ | H | 1 | $C_2H_5$ |
| $-CH_2C\equiv CH$ | H | 1 | $CH(CH_3)_2$ |
| $-CH_2C\equiv CH$ | $-CH_3$ | 1 | H |
| $-CH_2C\equiv CH$ | $-CF_3$ | 1 | H |
| $-CH_2C\equiv CH$ | $-Cl$ | 1 | H |
| $-CH_2C\equiv CH$ | $-COCH_3$ | 1 | H |
| $-CH_2C\equiv CH$ | $-COCCl_3$ | 1 | H |
| $-CH_2C\equiv CH$ | $-CH(OH)CCl_3$ | 1 | H |
| $-CH_2C\equiv CH$ | $-COOC_2H_5$ | 1 | H |
| $-CH_2C\equiv CH$ | -COO-Phenyl | 1 | H |
| $-CH_2C\equiv CH$ | -S-Phenyl | 1 | H |

Tabelle (Forts.)

| $R^1$ | X | n | $R^2$ |
|---|---|---|---|
| $-CH_2C{\equiv}CH$ | $-CH_2$-S-Phenyl | 1 | H |
| $-CH_2C{\equiv}CH$ | -CO-Phenyl | 1 | H |
| $-CH_2C{\equiv}CH$ | -Phenyl | 1 | H |
| $-CH_2C{\equiv}CH$ | $-S-(CH_2)_3CH_3$ | 1 | H |
| $-CH_2-CF_2Br$ | H | 1 | $CH_3$ |
| $-CH_2-CF_2Br$ | H | 1 | $C_2H_5$ |
| $-CH_2-CF_2Br$ | H | 1 | $CH(CH_3)_2$ |
| $-CH_2-CF_2Br$ | $-CH_3$ | 1 | H |
| $-CH_2-CF_2Br$ | $-CF_3$ | 1 | H |
| $-CH_2-CF_2Br$ | -Cl | 1 | H |
| $-CH_2-CF_2Br$ | $-COCH_3$ | 1 | H |
| $-CH_2-CF_2Br$ | $-COCCl_3$ | 1 | H |
| $-CH_2-CF_2Br$ | $-CH(OH)CCl_3$ | 1 | H |
| $-CH_2-CF_2Br$ | $-COOC_2H_5$ | 1 | H |
| $-CH_2-CF_2Br$ | -COO-Phenyl | 1 | H |
| $-CH_2-CF_2Br$ | -S-Phenyl | 1 | H |
| $-CH_2-CF_2Br$ | $-CH_2$-S-Phenyl | 1 | H |
| $-CH_2-CF_2Br$ | -CO-Phenyl | 1 | H |
| $-CH_2-CF_2Br$ | -Phenyl | 1 | H |
| $-CH_2-CF_2Br$ | $-S-(CH_2)_3CH_3$ | 1 | H |
| $-CH_2-C(Cl)=CH_2$ | H | 1 | $C_2H_5$ |
| $-CH_2-C(Cl)=CH_2$ | H | 1 | $CH(CH_3)_2$ |
| $-CH_2-C(Cl)=CH_2$ | $-CH_3$ | 1 | H |
| $-CH_2-C(Cl)=CH_2$ | $-CF_3$ | 1 | H |

Tabelle (Forts.)

| $R^1$ | X | n | $R^2$ |
|---|---|---|---|
| $-CH_2-C(Cl)=CH_2$ | $-Cl$ | 1 | H |
| $-CH_2-C(Cl)=CH_2$ | $-COCH_3$ | 1 | H |
| $-CH_2-C(Cl)=CH_2$ | $-COCCl_3$ | 1 | H |
| $-CH_2-C(Cl)=CH_2$ | $-CH(OH)CCl_3$ | 1 | H |
| $-CH_2-C(Cl)=CH_2$ | $-COOC_2H_5$ | 1 | H |
| $-CH_2-C(Cl)=CH_2$ | -COO-Phenyl | 1 | H |
| $-CH_2-C(Cl)=CH_2$ | -S-Phenyl | 1 | H |
| $-CH_2-C(Cl)=CH_2$ | $-CH_2$-S-Phenyl | 1 | H |
| $-CH_2-C(Cl)=CH_2$ | -CO-Phenyl | 1 | H |
| $-CH_2-C(Cl)=CH_2$ | -Phenyl | 1 | H |
| $-CH_2-C(Cl)=CH_2$ | $-S-(CH_2)_3CH_3$ | 1 | H |
| $-CH_2$-Cyclopropyl | H | 1 | $CH_3$ |
| $-CH_2$-Cyclopropyl | H | 1 | $C_2H_5$ |
| $-CH_2$-Cyclopropyl | H | 1 | $CH(CH_3)_2$ |
| $-CH_2$-Cyclopropyl | $-CH_3$ | 1 | H |
| $-CH_2$-Cyclopropyl | $-CF_3$ | 1 | H |
| $-CH_2$-Cyclopropyl | $-Cl$ | 1 | H |
| $-CH_2$-Cyclopropyl | $-COCH_3$ | 1 | H |
| $-CH_2$-Cyclopropyl | $-COCCl_3$ | 1 | H |
| $-CH_2$-Cyclopropyl | $-CH(OH)CCl_3$ | 1 | H |
| $-CH_2$-Cyclopropyl | $-COOC_2H_5$ | 1 | H |
| $-CH_2$-Cyclopropyl | -COO-Phenyl | 1 | H |
| $-CH_2$-Cyclopropyl | -S-Phenyl | 1 | H |
| $-CH_2$-Cyclopropyl | $-CH_2$-S-Phenyl | 1 | H |

Tabelle (Forts.)

| $R^1$ | X | n | $R^2$ |
|---|---|---|---|
| $-CH_2$-Cyclopropyl | -CO-Phenyl | 1 | H |
| $-CH_2$-Cyclopropyl | -Phenyl | 1 | H |
| $-CH_2$-Cyclopropyl | $-S-(CH_2)_3CH_3$ | 1 | H |
| $-CH_2-CH_2-OC_2H_5$ | H | 1 | $C_2H_5$ |
| $-CH_2-CH_2-OC_2H_5$ | H | 1 | $CH(CH_3)_2$ |
| $-CH_2-CH_2-OC_2H_5$ | $-CH_3$ | 1 | H |
| $-CH_2-CH_2-OC_2H_5$ | $-CF_3$ | 1 | H |
| $-CH_2-CH_2-OC_2H_5$ | -Cl | 1 | H |
| $-CH_2-CH_2-OC_2H_5$ | $-COCH_3$ | 1 | H |
| $-CH_2-CH_2-OC_2H_5$ | $-COCCl_3$ | 1 | H |
| $-CH_2-CH_2-OC_2H_5$ | $-CH(OH)CCl_3$ | 1 | H |
| $-CH_2-CH_2-OC_2H_5$ | $-COOC_2H_5$ | 1 | H |
| $-CH_2-CH_2-OC_2H_5$ | -COO-Phenyl | 1 | H |
| $-CH_2-CH_2-OC_2H_5$ | -S-Phenyl | 1 | H |
| $-CH_2-CH_2-OC_2H_5$ | $-CH_2$-S-Phenyl | 1 | H |
| $-CH_2-CH_2-OC_2H_5$ | -CO-Phenyl | 1 | H |
| $-CH_2-CH_2-OC_2H_5$ | -Phenyl | 1 | H |
| $-CH_2-CH_2-OC_2H_5$ | $-S-(CH_2)_3CH_3$ | 1 | H |
| $-CH_2-CH_2-O-CH_2CH_2Cl$ | H | 1 | $CH_3$ |
| $-CH_2-CH_2-O-CH_2CH_2Cl$ | H | 1 | $C_2H_5$ |
| $-CH_2-CH_2-O-CH_2CH_2Cl$ | H | 1 | $CH(CH_3)_2$ |
| $-CH_2-CH_2-O-CH_2CH_2Cl$ | $-CH_3$ | 1 | H |
| $-CH_2-CH_2-O-CH_2CH_2Cl$ | $-CF_3$ | 1 | H |
| $-CH_2-CH_2-O-CH_2CH_2Cl$ | -Cl | 1 | H |

Tabelle (Forts.)

| $R^1$ | X | n | $R^2$ |
|---|---|---|---|
| $-CH_2-CH_2-O-CH_2CH_2Cl$ | $-COCH_3$ | 1 | H |
| $-CH_2-CH_2-O-CH_2CH_2Cl$ | $-COCCl_3$ | 1 | H |
| $-CH_2-CH_2-O-CH_2CH_2Cl$ | $-CH(OH)CCl_3$ | 1 | H |
| $-CH_2-CH_2-O-CH_2CH_2Cl$ | $-COOC_2H_5$ | 1 | H |
| $-CH_2-CH_2-O-CH_2CH_2Cl$ | -COO-Phenyl | 1 | H |
| $-CH_2-CH_2-O-CH_2CH_2Cl$ | -S-Phenyl | 1 | H |
| $-CH_2-CH_2-O-CH_2CH_2Cl$ | $-CH_2$-S-Phenyl | 1 | H |
| $-CH_2-CH_2-O-CH_2CH_2Cl$ | -CO-Phenyl | 1 | H |
| $-CH_2-CH_2-O-CH_2CH_2Cl$ | -Phenyl | 1 | H |
| $-CH_2-CH_2-O-CH_2CH_2Cl$ | $-S-(CH_2)_3CH_3$ | 1 | H |
| $-CH_2-CH_2-N(CH_3)_2$ | H | 1 | $CH_3$ |
| $-CH_2-CH_2-N(CH_3)_2$ | H | 1 | $C_2H_5$ |
| $-CH_2-CH_2-N(CH_3)_2$ | H | 1 | $CH(CH_3)_2$ |
| $-CH_2-CH_2-N(CH_3)_2$ | $-CH_3$ | 1 | H |
| $-CH_2-CH_2-N(CH_3)_2$ | $-CF_3$ | 1 | H |
| $-CH_2-CH_2-N(CH_3)_2$ | -Cl | 1 | H |
| $-CH_2-CH_2-N(CH_3)_2$ | $-COCH_3$ | 1 | H |
| $-CH_2-CH_2-N(CH_3)_2$ | $-COCCl_3$ | 1 | H |
| $-CH_2-CH_2-N(CH_3)_2$ | $-CH(OH)CCl_3$ | 1 | H |
| $-CH_2-CH_2-N(CH_3)_2$ | $-COOC_2H_5$ | 1 | H |
| $-CH_2-CH_2-N(CH_3)_2$ | -COO-Phenyl | 1 | H |
| $-CH_2-CH_2-N(CH_3)_2$ | -S-Phenyl | 1 | H |
| $-CH_2-CH_2-N(CH_3)_2$ | $-CH_2$-S-Phenyl | 1 | H |
| $-CH_2-CH_2-N(CH_3)_2$ | -CO-Phenyl | 1 | H |

Tabelle (Forts.)

| $R^1$ | X | n | $R^2$ |
|---|---|---|---|
| $-CH_2-CH_2-N(CH_3)_2$ | -Phenyl | 1 | H |
| $-CH_2-CH_2-N(CH_3)_2$ | $-S-(CH_2)_3CH_3$ | 1 | H |
| $-CH_2-CH_2-S-CH_3$ | H | 1 | $CH_3$ |
| $-CH_2-CH_2-S-CH_3$ | H | 1 | $C_2H_5$ |
| $-CH_2-CH_2-S-CH_3$ | H | 1 | $CH(CH_3)_2$ |
| $-CH_2-CH_2-S-CH_3$ | $-CH_3$ | 1 | H |
| $-CH_2-CH_2-S-CH_3$ | $-CF_3$ | 1 | H |
| $-CH_2-CH_2-S-CH_3$ | -Cl | 1 | H |
| $-CH_2-CH_2-S-CH_3$ | $-COCH_3$ | 1 | H |
| $-CH_2-CH_2-S-CH_3$ | $-COCCl_3$ | 1 | H |
| $-CH_2-CH_2-S-CH_3$ | $-CH(OH)CCl_3$ | 1 | H |
| $-CH_2-CH_2-S-CH_3$ | $-COOC_2H_5$ | 1 | H |
| $-CH_2-CH_2-S-CH_3$ | -COO-Phenyl | 1 | H |
| $-CH_2-CH_2-S-CH_3$ | -S-Phenyl | 1 | H |
| $-CH_2-CH_2-S-CH_3$ | $-CH_2$-S-Phenyl | 1 | H |
| $-CH_2-CH_2-S-CH_3$ | -CO-Phenyl | 1 | H |
| $-CH_2-CH_2-S-CH_3$ | -Phenyl | 1 | H |
| $-CH_2-CH_2-S-CH_3$ | $-S-(CH_2)_3CH_3$ | 1 | H |
| $-CH_2-CH_2-S-CH_2F$ | H | 1 | $CH_3$ |
| $-CH_2-CH_2-S-CH_2F$ | H | 1 | $C_2H_5$ |
| $-CH_2-CH_2-S-CH_2F$ | H | 1 | $CH(CH_3)_2$ |
| $-CH_2-CH_2-S-CH_2F$ | $-CH_3$ | 1 | H |
| $-CH_2-CH_2-S-CH_2F$ | $-CF_3$ | 1 | H |
| $-CH_2-CH_2-S-CH_2F$ | -Cl | 1 | H |

Tabelle (Forts.)

| $R^1$ | X | n | $R^2$ |
|---|---|---|---|
| $-CH_2-CH_2-S-CH_2F$ | $-COCH_3$ | 1 | H |
| $-CH_2-CH_2-S-CH_2F$ | $-COCCl_3$ | 1 | H |
| $-CH_2-CH_2-S-CH_2F$ | $-CH(OH)CCl_3$ | 1 | H |
| $-CH_2-CH_2-S-CH_2F$ | $-COOC_2H_5$ | 1 | H |
| $-CH_2-CH_2-S-CH_2F$ | -COO-Phenyl | 1 | H |
| $-CH_2-CH_2-S-CH_2F$ | -S-Phenyl | 1 | H |
| $-CH_2-CH_2-S-CH_2F$ | $-CH_2$-S-Phenyl | 1 | H |
| $-CH_2-CH_2-S-CH_2F$ | -CO-Phenyl | 1 | H |
| $-CH_2-CH_2-S-CH_2F$ | -Phenyl | 1 | H |
| $-CH_2-CH_2-S-CH_2F$ | $-S-(CH_2)_3CH_3$ | 1 | H |
| $-CH_2$-Phenyl | H | 1 | $CH_3$ |
| $-CH_2$-Phenyl | H | 1 | $C_2H_5$ |
| $-CH_2$-Phenyl | H | 1 | $CH(CH_3)_2$ |
| $-CH_2$-Phenyl | $-CH_3$ | 1 | H |
| $-CH_2$-Phenyl | $-CF_3$ | 1 | H |
| $-CH_2$-Phenyl | -Cl | 1 | H |
| $-CH_2$-Phenyl | $-COCH_3$ | 1 | H |
| $-CH_2$-Phenyl | $-COCCl_3$ | 1 | H |
| $-CH_2$-Phenyl | $-CH(OH)CCl_3$ | 1 | H |
| $-CH_2$-Phenyl | $-COOC_2H_5$ | 1 | H |
| $-CH_2$-Phenyl | -COO-Phenyl | 1 | H |
| $-CH_2$-Phenyl | -S-Phenyl | 1 | H |
| $-CH_2$-Phenyl | $-CH_2$-S-Phenyl | 1 | H |
| $-CH_2$-Phenyl | -CO-Phenyl | 1 | H |

Tabelle (Forts.)

| $R^1$ | X | n | $R^2$ |
|---|---|---|---|
| $-CH_2$-Phenyl | -Phenyl | 1 | H |
| $-CH_2$-Phenyl | $-S-(CH_2)_3CH_3$ | 1 | H |
| $-CH_2$-[4-Cl-Phenyl] | H | 1 | $CH_3$ |
| $-CH_2$-[4-Cl-Phenyl] | H | 1 | $C_2H_5$ |
| $-CH_2$-[4-Cl-Phenyl] | H | 1 | $CH(CH_3)_2$ |
| $-CH_2$-[4-Cl-Phenyl] | $-CH_3$ | 1 | H |
| $-CH_2$-[4-Cl-Phenyl] | $-CF_3$ | 1 | H |
| $-CH_2$-[4-Cl-Phenyl] | -Cl | 1 | H |
| $-CH_2$-[4-Cl-Phenyl] | $-COCH_3$ | 1 | H |
| $-CH_2$-[4-Cl-Phenyl] | $-COCCl_3$ | 1 | H |
| $-CH_2$-[4-Cl-Phenyl] | $-CH(OH)CCl_3$ | 1 | H |
| $-CH_2$-[4-Cl-Phenyl] | $-COOC_2H_5$ | 1 | H |
| $-CH_2$-[4-Cl-Phenyl] | -COO-Phenyl | 1 | H |
| $-CH_2$-[4-Cl-Phenyl] | -S-Phenyl | 1 | H |
| $-CH_2$-[4-Cl-Phenyl] | $-CH_2$-S-Phenyl | 1 | H |
| $-CH_2$-[4-Cl-Phenyl] | -CO-Phenyl | 1 | H |
| $-CH_2$-[4-Cl-Phenyl] | -Phenyl | 1 | H |
| $-CH_2$-[4-Cl-Phenyl] | $-S-(CH_2)_3CH_3$ | 1 | H |
| $-CH_2-CH=CH-CH_2$-[4-F-Phenyl] | H | 1 | $CH_3$ |
| $-CH_2-CH=CH-CH_2$-[4-F-Phenyl] | H | 1 | $C_2H_5$ |
| $-CH_2-CH=CH-CH_2$-[4-F-Phenyl] | H | 1 | $CH(CH_3)_2$ |
| $-CH_2-CH=CH-CH_2$-[4-F-Phenyl] | $-CH_3$ | 1 | H |
| $-CH_2-CH=CH-CH_2$-[4-F-Phenyl] | $-CF_3$ | 1 | H |
| $-CH_2-CH=CH-CH_2$-[4-F-Phenyl] | -Cl | 1 | H |

Tabelle (Forts.)

| $R^1$ | X | n | $R^2$ |
|---|---|---|---|
| $-CH_2-CH=CH-CH_2-$[4-F-Phenyl] | $-COCH_3$ | 1 | H |
| $-CH_2-CH=CH-CH_2-$[4-F-Phenyl] | $-COCCl_3$ | 1 | H |
| $-CH_2-CH=CH-CH_2-$[4-F-Phenyl] | $-CH(OH)CCl_3$ | 1 | H |
| $-CH_2-CH=CH-CH_2-$[4-F-Phenyl] | $-COOC_2H_5$ | 1 | H |
| $-CH_2-CH=CH-CH_2-$[4-F-Phenyl] | -COO-Phenyl | 1 | H |
| $-CH_2-CH=CH-CH_2-$[4-F-Phenyl] | -S-Phenyl | 1 | H |
| $-CH_2-CH=CH-CH_2-$[4-F-Phenyl] | $-CH_2$-S-Phenyl | 1 | H |
| $-CH_2-CH=CH-CH_2-$[4-F-Phenyl] | -CO-Phenyl | 1 | H |
| $-CH_2-CH=CH-CH_2-$[4-F-Phenyl] | -Phenyl | 1 | H |
| $-CH_2-CH=CH-CH_2-$[4-F-Phenyl] | $-S-(CH_2)_3CH_3$ | 1 | H |
| $-CH_2-(CH_2)_2$-O-Phenyl | H | 1 | $CH_3$ |
| $-CH_2-(CH_2)_2$-O-Phenyl | H | 1 | $C_2H_5$ |
| $-CH_2-(CH_2)_2$-O-Phenyl | H | 1 | $CH(CH_3)_2$ |
| $-CH_2-(CH_2)_2$-O-Phenyl | $-CH_3$ | 1 | H |
| $-CH_2-(CH_2)_2$-O-Phenyl | $-CF_3$ | 1 | H |
| $-CH_2-(CH_2)_2$-O-Phenyl | -Cl | 1 | H |
| $-CH_2-(CH_2)_2$-O-Phenyl | $-COCH_3$ | 1 | H |
| $-CH_2-(CH_2)_2$-O-Phenyl | $-COCCl_3$ | 1 | H |
| $-CH_2-(CH_2)_2$-O-Phenyl | $-CH(OH)CCl_3$ | 1 | H |
| $-CH_2-(CH_2)_2$-O-Phenyl | $-COOC_2H_5$ | 1 | H |
| $-CH_2-(CH_2)_2$-O-Phenyl | -COO-Phenyl | 1 | H |
| $-CH_2-(CH_2)_2$-O-Phenyl | -S-Phenyl | 1 | H |
| $-CH_2-(CH_2)_2$-O-Phenyl | $-CH_2$-S-Phenyl | 1 | H |
| $-CH_2-(CH_2)_2$-O-Phenyl | -CO-Phenyl | 1 | H |

Tabelle (Forts.)

| $R^1$ | X | n | $R^2$ |
|---|---|---|---|
| $-CH_2-(CH_2)_2-O-$Phenyl | -Phenyl | 1 | H |
| $-CH_2-(CH_2)_2-O-$Phenyl | $-S-(CH_2)_3CH_3$ | 1 | H |
| 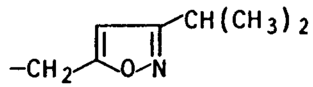 | H | 1 | $CH_3$ |
| 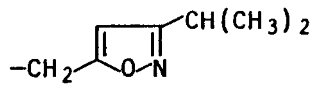 | H | 1 | $C_3H_5$ |
| 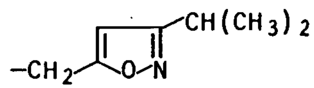 | H | 1 | $CH(CH_3)_2$ |
| 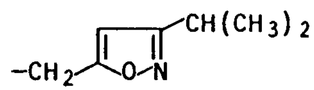 | $-CH_3$ | 1 | H |
| 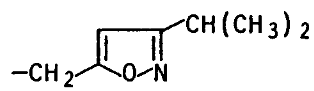 | $-CF_3$ | 1 | H |

Tabelle (Forts.)

| $R^1$ | X | n | $R^2$ |
|---|---|---|---|
| 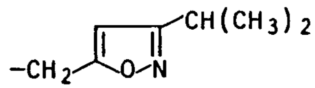 | $-Cl$ | 1 | H |
| 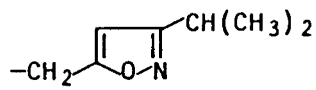 | $-COCH_3$ | 1 | H |
| 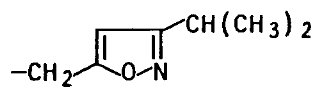 | $-COCCl_3$ | 1 | H |
| 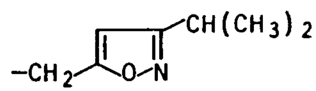 | $-CH(OH)CCl_3$ | 1 | H |
| 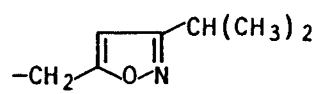 | $-COOC_2H_5$ | 1 | H |
| 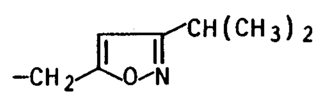 | $-COO-Phenyl$ | 1 | H |

Tabelle (Forts.)

| $R^1$ | X | n | $R^2$ |
|---|---|---|---|
| 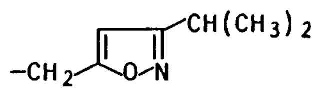 | $-S-Phenyl$ | 1 | H |
| 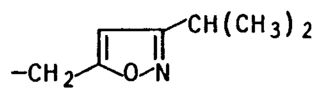 | $-CH_2-S-Phenyl$ | 1 | H |
| 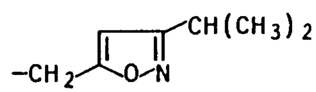 | $-CO-Phenyl$ | 1 | H |
| 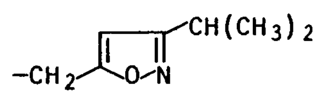 | $-Phenyl$ | 1 | H |
| 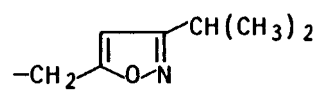 | $-S-(CH_2)_3CH_3$ | 1 | H |
| 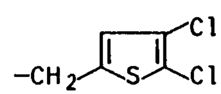 | H | 1 | $CH_3$ |

Tabelle (Forts.)

| $R^1$ | X | n | $R^2$ |
|---|---|---|---|
| 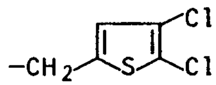 | H | 1 | $C_2H_5$ |
| 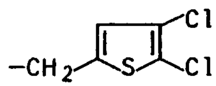 | H | 1 | $CH(CH_3)_2$ |
| 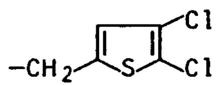 | $-CH_3$ | 1 | H |
| 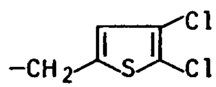 | $-CF_3$ | 1 | H |
| 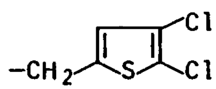 | $-Cl$ | 1 | H |
| 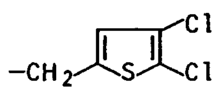 | $-COCH_3$ | 1 | H |
| 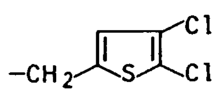 | $-COCCl_3$ | 1 | H |

Tabelle (Forts.)

| $R^1$ | X | n | $R^2$ |
|---|---|---|---|
| 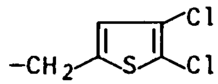 | $-CH(OH)CCl_3$ | 1 | H |
| 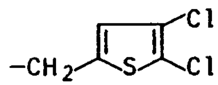 | $-COOC_2H_5$ | 1 | H |
| 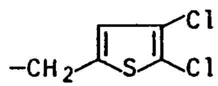 | -COO-Phenyl | 1 | H |
| 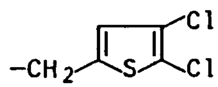 | -S-Phenyl | 1 | H |
| 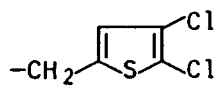 | $-CH_2$-S-Phenyl | 1 | H |
| 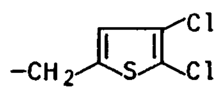 | -CO-Phenyl | 1 | H |
| 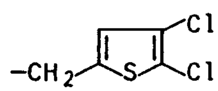 | -Phenyl | 1 | H |

Tabelle (Forts.)

| $R^1$ | X | n | $R^2$ |
| --- | --- | --- | --- |
| 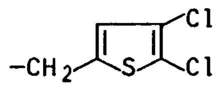 | $-S-(CH_2)_3CH_3$ | 1 | H |
| 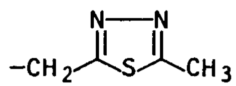 | H | 1 | $CH_3$ |
| 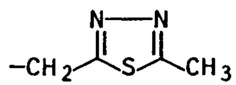 | H | 1 | $C_2H_5$ |
| 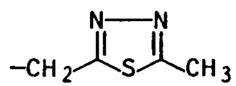 | H | 1 | $CH(CH_3)_2$ |
| 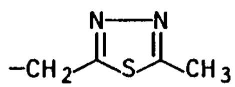 | $-CH_3$ | 1 | H |
| 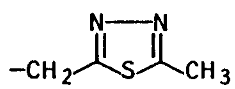 | $-CF_3$ | 1 | H |
| 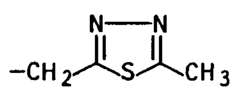 | $-Cl$ | 1 | H |

Tabelle (Forts.)

| $R^1$ | X | n | $R^2$ |
|---|---|---|---|
| 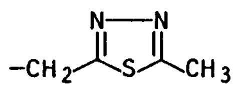 | $-COCH_3$ | 1 | H |
| 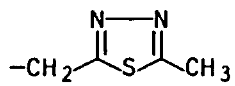 | $-COCCl_3$ | 1 | H |
| 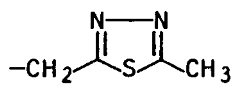 | $-CH(OH)CCl_3$ | 1 | H |
| 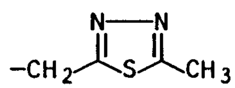 | $-COOC_2H_5$ | 1 | H |
| 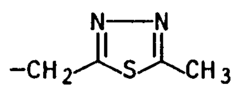 | -COO-Phenyl | 1 | H |
| 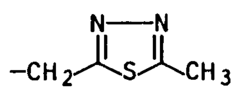 | -S-Phenyl | 1 | H |
| 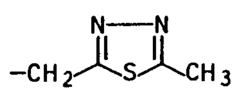 | $-CH_2$-2-Phenyl | 1 | H |

Tabelle (Forts.)

| $R^1$ | X | n | $R^2$ |
|---|---|---|---|
| 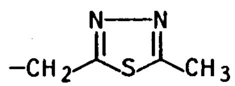 | $-CO-$Phenyl | 1 | H |
| 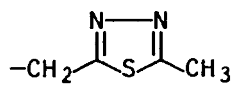 | $-$Phenyl | 1 | H |
| 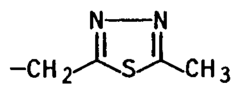 | $-S-(CH_2)_3CH_3$ | 1 | H |
| 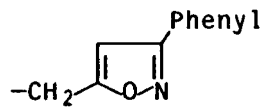 | H | 1 | $CH_3$ |
| 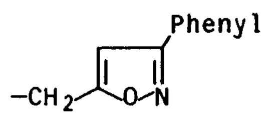 | H | 1 | $C_2H_5$ |
| 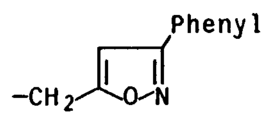 | H | 1 | $CH(CH_3)_2$ |
| 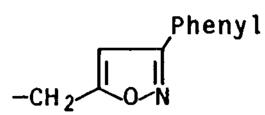 | $-CH_3$ | 1 | H |

Tabelle (Forts.)

| $R^1$ | X | n | $R^2$ |
|---|---|---|---|
| 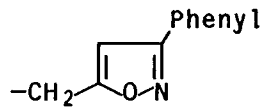 | $-CF_3$ | 1 | H |
| 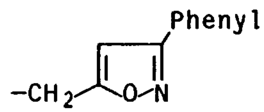 | $-Cl$ | 1 | H |
| 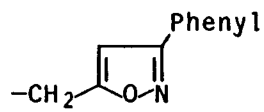 | $-COCH_3$ | 1 | H |
| 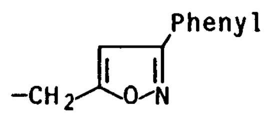 | $-COCCl_3$ | 1 | H |
| 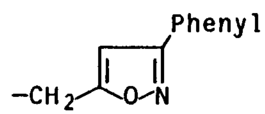 | $-CH(OH)CCl_3$ | 1 | H |
| 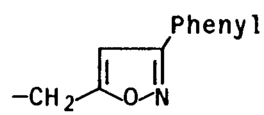 | $-COOC_2H_5$ | 1 | H |

Tabelle (Forts.)

| $R^1$ | X | n | $R^2$ |
|---|---|---|---|
| 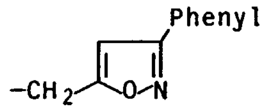 | $-COO-$Phenyl | 1 | H |
| 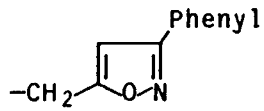 | $-S-$Phenyl | 1 | H |
| 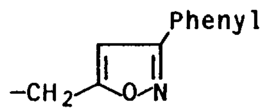 | $-CH_2-S-$Phenyl | 1 | H |
| 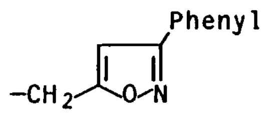 | $-CO-$Phenyl | 1 | H |
| 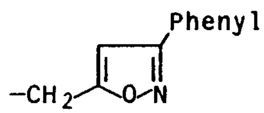 | $-$Phenyl | 1 | H |
| 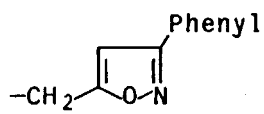 | $-S-(CH_2)_3CH_3$ | 1 | H |

Tabelle (Forts.)

| $R^1$ | X | n | $R^2$ |
|---|---|---|---|
| 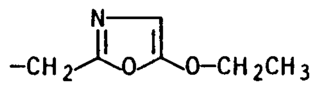 | H | 1 | $CH_3$ |
| 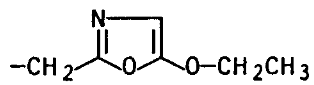 | H | 1 | $C_2H_5$ |
| 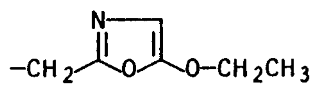 | H | 1 | $CH(CH_3)_2$ |
| 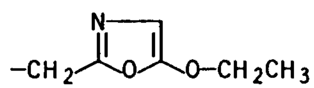 | $-CH_3$ | 1 | H |
| 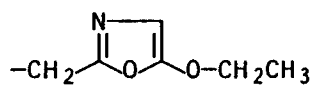 | $-CF_3$ | 1 | H |
| 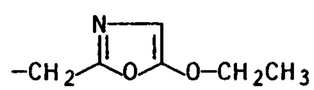 | $-Cl$ | 1 | H |
| 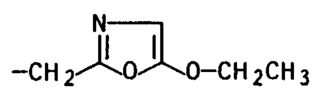 | $-COCH_3$ | 1 | H |

Tabelle (Forts.)

| $R^1$ | X | n | $R^2$ |
|---|---|---|---|
| 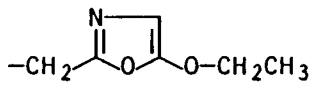 | $-COCCl_3$ | 1 | H |
| 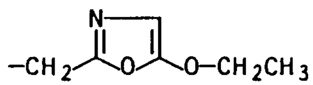 | $-CH(OH)CCl_3$ | 1 | H |
| 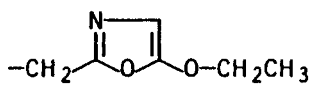 | $-COOC_2H_5$ | 1 | H |
| 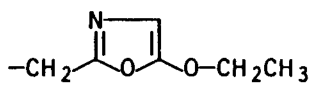 | -COO-Phenyl | 1 | H |
| 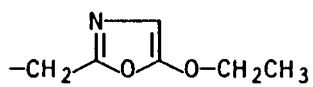 | -S-Phenyl | 1 | H |
| 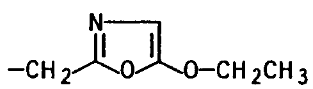 | $-CH_2$-S-Phenyl | 1 | H |
| 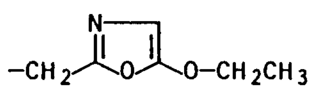 | -CO-Phenyl | 1 | H |

Tabelle (Forts.)

| $R^1$ | X | n | $R^2$ |
|---|---|---|---|
| 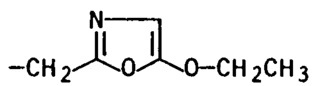 | -Phenyl | 1 | H |
| 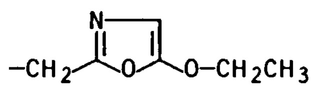 | $-S-(CH_2)_3CH_3$ | 1 | H |
| 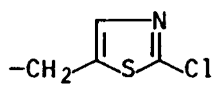 | H | 1 | $CH_3$ |
| 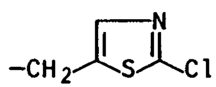 | H | 1 | $C_2H_5$ |
| 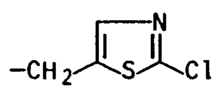 | H | 1 | $CH(CH_3)_2$ |
| 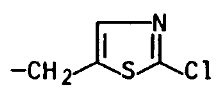 | $-CH_3$ | 1 | H |
| 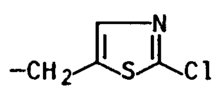 | $-CF_3$ | 1 | H |

Tabelle (Forts.)

| $R^1$ | X | n | $R^2$ |
|---|---|---|---|
| 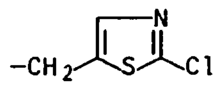 | -Cl | 1 | H |
| 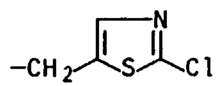 | $-COCH_3$ | 1 | H |
| 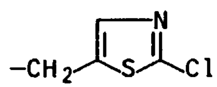 | $-COCCl_3$ | 1 | H |
| 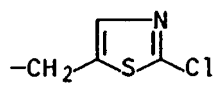 | $-CH(OH)CCl_3$ | 1 | H |
| 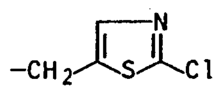 | $-COOC_2H_5$ | 1 | H |
| 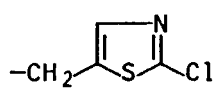 | -COO-Phenyl | 1 | H |
| 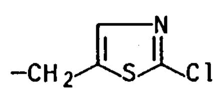 | -S-Phenyl | 1 | H |

Tabelle (Forts.)

| $R^1$ | X | n | $R^2$ |
| --- | --- | --- | --- |
| 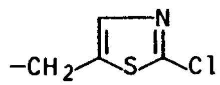 | $-CH_2-S-$Phenyl | 1 | H |
| 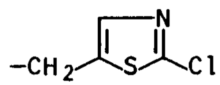 | -CO-Phenyl | 1 | H |
| 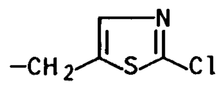 | -Phenyl | 1 | H |
| 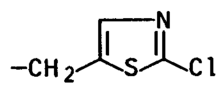 | $-S-(CH_2)_3CH_3$ | 1 | H |
| 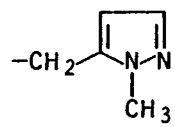 | H | 1 | $CH_3$ |
| 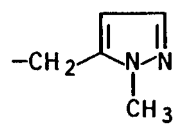 | H | 1 | $C_2H_5$ |
| 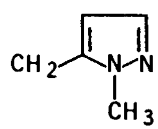 | H | 1 | $CH(CH_3)_2$ |

Tabelle (Forts.)

| $R^1$ | X | n | $R^2$ |
|---|---|---|---|
| 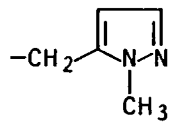 | $-CH_3$ | 1 | H |
| 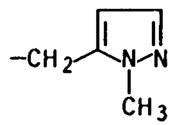 | $-CF_3$ | 1 | H |
| 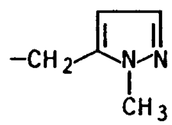 | -Cl | 1 | H |
| 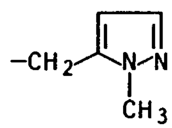 | $-COCH_3$ | 1 | H |
| 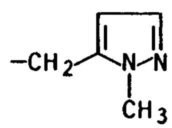 | $-COCCl_3$ | 1 | H |
| 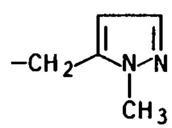 | $-CH(OH)CCl_3$ | 1 | H |

Tabelle (Forts.)

| $R^1$ | X | n | $R^2$ |
|---|---|---|---|
| 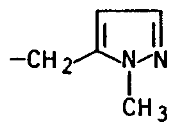 | $-COOC_2H_5$ | 1 | H |
| 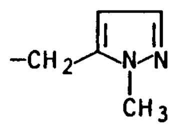 | -COO-Phenyl | 1 | H |
| 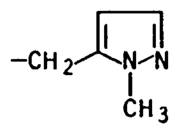 | -S-Phenyl | 1 | H |
| 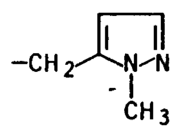 | $-CH_2$-S-Phenyl | 1 | H |
| 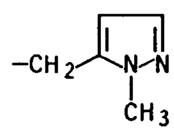 | -CO-Phenyl | 1 | H |
| 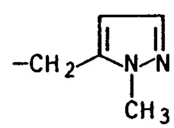 | -Phenyl | 1 | H |

Tabelle (Forts.)

| $R^1$ | X | n | $R^2$ |
|---|---|---|---|
| 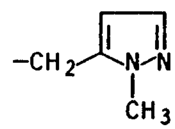 | $-S-(CH_2)_3CH_3$ | 1 | H |
| 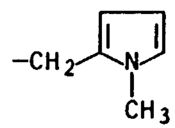 | H | 1 | $CH_3$ |
| 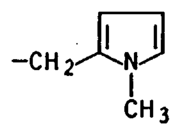 | H | 1 | $C_2H_5$ |
| 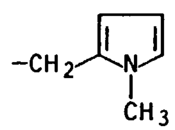 | H | 1 | $CH(CH_3)_2$ |
| 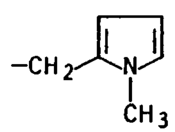 | $-CH_3$ | 1 | H |
| 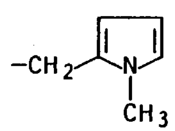 | $-CF_3$ | 1 | H |

Tabelle (Forts.)

| $R^1$ | X | n | $R^2$ |
|---|---|---|---|
| 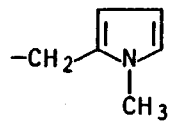 | $-Cl$ | 1 | H |
| 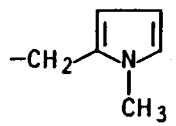 | $-COCH_3$ | 1 | H |
| 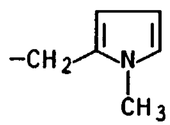 | $-COCCl_3$ | 1 | H |
| 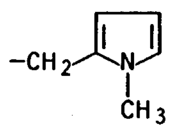 | $-CH(OH)CCl_3$ | 1 | H |
| 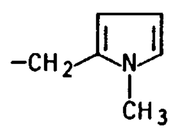 | $-COOC_2H_5$ | 1 | H |
| 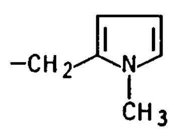 | -COO-Phenyl | 1 | H |

Tabelle (Forts.)

| $R^1$ | X | n | $R^2$ |
|---|---|---|---|
| 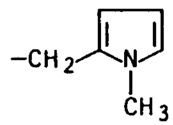 | $-S-Phenyl$ | 1 | H |
| 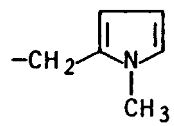 | $-CH_2-S-Phenyl$ | 1 | H |
| 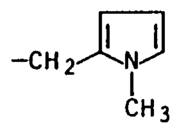 | $-CO-Phenyl$ | 1 | H |
| 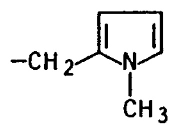 | $-Phenyl$ | 1 | H |
| 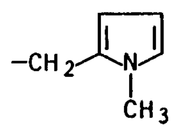 | $-S-(CH_2)_3CH_3$ | 1 | H |
| 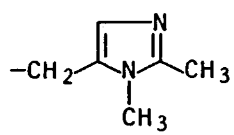 | H | 1 | $CH_3$ |

Tabelle (Forts.)

| $R^1$ | X | n | $R^2$ |
|---|---|---|---|
| 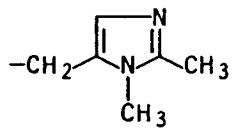 | H | 1 | $C_2H_5$ |
| 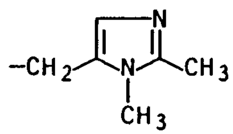 | H | 1 | $CH(CH_3)_2$ |
| 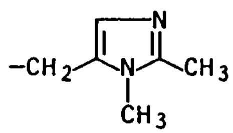 | $-CH_3$ | 1 | H |
| 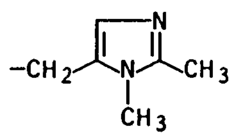 | $-CF_3$ | 1 | H |
| 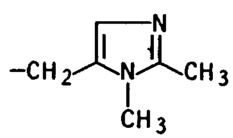 | -Cl | 1 | H |
| 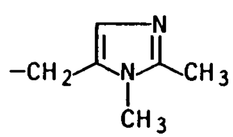 | $-COCH_3$ | 1 | H |

Tabelle (Forts.)

| $R^1$ | X | n | $R^2$ |
|---|---|---|---|
| 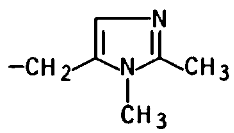 | $-COCCl_3$ | 1 | H |
| 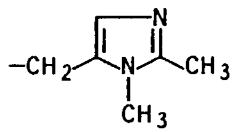 | $-CH(OH)CCl_3$ | 1 | H |
| 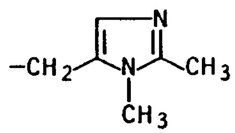 | $-COOC_2H_5$ | 1 | H |
| 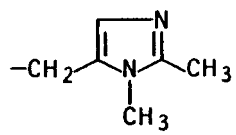 | -COO-Phenyl | 1 | H |
| 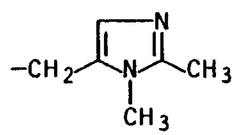 | -S-Phenyl | 1 | H |
| 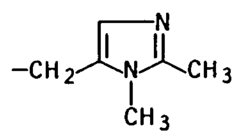 | $-CH_2$-S-Phenyl | 1 | H |

Tabelle (Forts.)

| $R^1$ | X | n | $R^2$ |
|---|---|---|---|
| 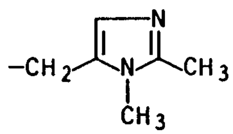 | -CO-Phenyl | 1 | H |
| 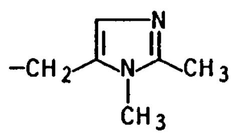 | -Phenyl | 1 | H |
| 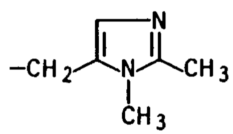 | $-S-(CH_2)_3CH_3$ | 1 | H |
| 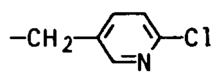 | H | 1 | $CH_3$ |
| 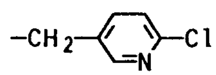 | H | 1 | $C_2H_5$ |
| 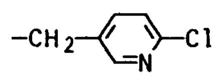 | H | 1 | $CH(CH_3)_2$ |
| 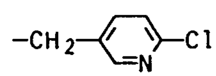 | $-CH_3$ | 1 | H |

Tabelle (Forts.)

| $R^1$ | X | n | $R^2$ |
|---|---|---|---|
| 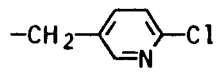 | $-CF_3$ | 1 | H |
| 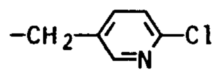 | -Cl | 1 | H |
| 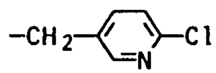 | $-COCH_5$ | 1 | H |
| 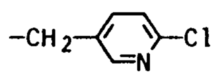 | $-COCCl_3$ | 1 | H |
| 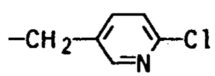 | $-CH(OH)CCl_3$ | 1 | H |
| 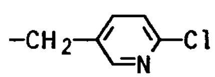 | $-COOC_2H_5$ | 1 | H |
| 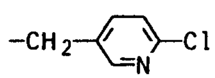 | -COO-Phenyl | 1 | H |

Tabelle (Forts.)

| $R^1$ | X | n | $R^2$ |
|---|---|---|---|
| 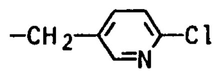 | -S-Phenyl | 1 | H |
| 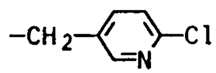 | $-CH_2$-S-Phenyl | 1 | H |
| 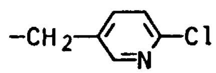 | -CO-Phenyl | 1 | H |
| 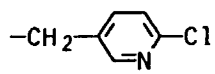 | -Phenyl | 1 | H |
| 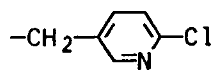 | $-S-(CH_2)_3CH_3$ | 1 | H |
| 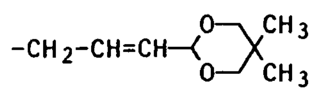 | H | 1 | $CH_3$ |
| 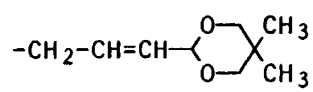 | H | 1 | $C_2H_5$ |

Tabelle (Forts.)

| $R^1$ | X | n | $R^2$ |
|---|---|---|---|
| 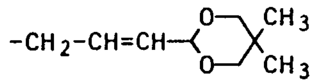 | H | 1 | $CH(CH_3)_2$ |
| 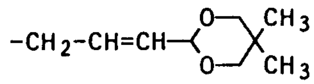 | $-CH_3$ | 1 | H |
| 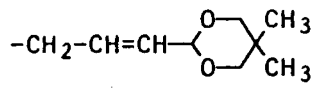 | $-CF_3$ | 1 | H |
| 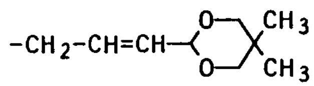 | -Cl | 1 | H |
| 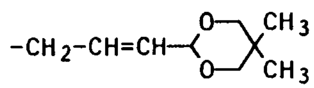 | $-COCH_3$ | 1 | H |
| 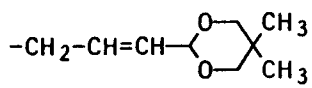 | $-COCCl_3$ | 1 | H |
| 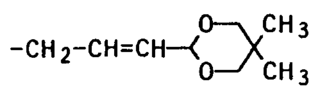 | $-CH(OH)CCl_3$ | 1 | H |

Tabelle (Forts.)

| $R^1$ | X | n | $R^2$ |
|---|---|---|---|
| 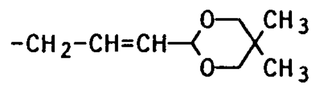 | $-COOC_2H_5$ | 1 | H |
| 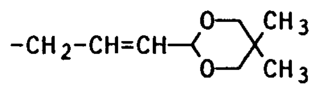 | -COO-Phenyl | 1 | H |
| 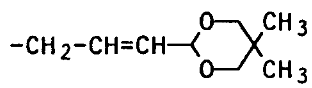 | -S-Phenyl | 1 | H |
| 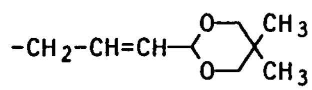 | $-CH_2$-S-Phenyl | 1 | H |
| 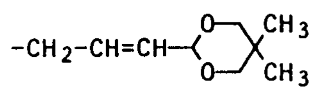 | -CO-Phenyl | 1 | H |
| 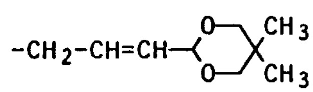 | -Phenyl | 1 | H |
| 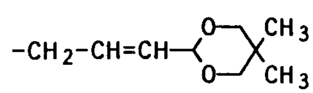 | $-S-(CH_2)_3CH_3$ | 1 | H |

Tabelle (Forts.)

| $R^1$ | X | n | $R^2$ |
|---|---|---|---|
| 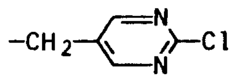 | H | 1 | $CH_3$ |
| 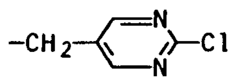 | H | 1 | $C_2H_5$ |
| 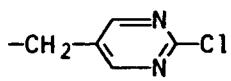 | H | 1 | $CH(CH_3)_2$ |
| 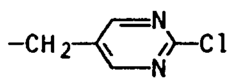 | $-CH_3$ | 1 | H |
| 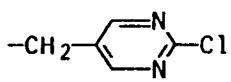 | $-CF_3$ | 1 | H |
| 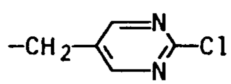 | -Cl | 1 | H |
| 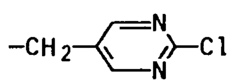 | $-COCH_3$ | 1 | H |

Tabelle (Forts.)

| $R^1$ | X | n | $R^2$ |
|---|---|---|---|
| 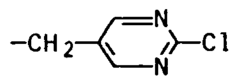 | $-COCCl_3$ | 1 | H |
| 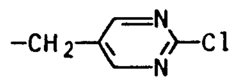 | $-CH(OH)CCl_3$ | 1 | H |
| 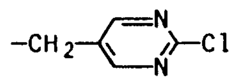 | $-COOC_2H_5$ | 1 | H |
| 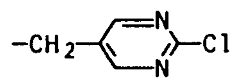 | -COO-Phenyl | 1 | H |
| 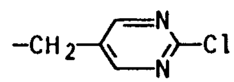 | -S-Phenyl | 1 | H |
| 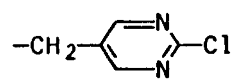 | $-CH_2$-S-Phenyl | 1 | H |
| 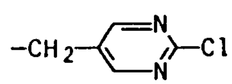 | -CO-Phenyl | 1 | H |

Tabelle (Forts.)

| $R^1$ | X | n | $R^2$ |
|---|---|---|---|
| 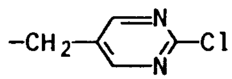 | -Phenyl | 1 | H |
| 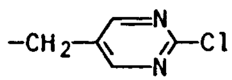 | $-S-(CH_2)_3CH_3$ | 1 | H |
| 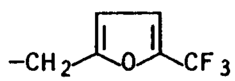 | H | 1 | $CH_3$ |
| 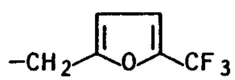 | H | 1 | $C_2H_5$ |
| 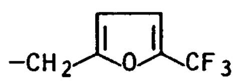 | H | 1 | $CH(CH_3)_2$ |
| 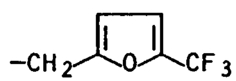 | $-CH_3$ | 1 | H |
| 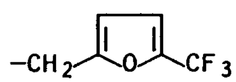 | $-CF_3$ | 1 | H |

Tabelle (Forts.)

| $R^1$ | X | n | $R^2$ |
|---|---|---|---|
| 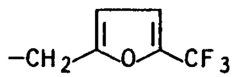 | $-Cl$ | 1 | H |
| 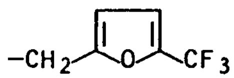 | $-COCH_3$ | 1 | H |
| 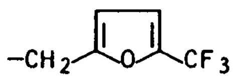 | $-COCCl_3$ | 1 | H |
| 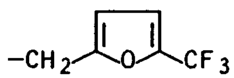 | $-CH(OH)CCl_3$ | 1 | H |
| 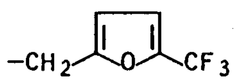 | $-COOC_2H_5$ | 1 | H |
| 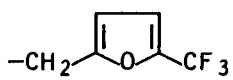 | -COO-Phenyl | 1 | H |
| 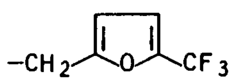 | -S-Phenyl | 1 | H |

Tabelle (Forts.)

| $R^1$ | X | n | $R^2$ |
|---|---|---|---|
| 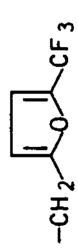 | $-CH_2-S-$Phenyl | 1 | H |
| 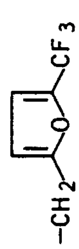 | $-CO-$Phenyl | 1 | H |
| 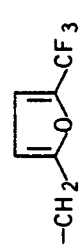 | -Phenyl | 1 | H |
| 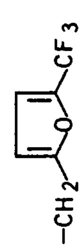 | $-S-(CH_2)_3CH_3$ | 1 | H |

Die Verbindungen der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticuiana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar,

Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyliopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:

I. 5 Gew.-Teile der Verbindung Nr. 2 werden mit 95 Gew.-Teilen feinteiligem Kaolin innig vermischt. Man erhält auf diese Weise ein Stäubemittel, das 5 Gew.% des Wirkstoffs enthält.

II. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).

III. 10 Gew.-Teile der Verbindung Nr. 6 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).

IV. 20 Gew.-Teile der Verbindung Nr. 1 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).

V. 80 Gew.-Teile der Verbindung Nr. 5 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle

vermahlen (Wirkstoffgehalt 80 Gew.%).

VI. Man vermischt 90 Gew.-Teile der Verbindung Nr. 4 mit 10 Gew.-Teilen N-Methyl-α-pyrrolidon und erhält eine Lösung, die zur Anwendung in Form kleinster Tropfen geeignet ist (Wirkstoffgehalt 90 Gew.%).

VII. 20 Gew.-Teile der Verbindung Nr. 12 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

VIII. 20 Gew.-Teile des Wirkstoffs Nr. 7 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 3, vorzugsweise 0,05 bis 1 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

## Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

### A. Herstellung der Zwischenprodukte

#### A1. Herstellung von 1-Amino-3-methoxyaminopropan Dihydrochlorid (II-1)

10 g N-(3-Brompropyl)-phthalimid (0,037 mol), 6,2 g Methoxylamin Hydrochlorid (0,075 mol) und 15,0 g Natriumcarbonat werden in 150 ml Ethanol suspendiert und 12 Stunden am Rückfluß erhitzt. Nach dem Abkühlen wird von den Salzen abfiltriert und das Lösungsmittel entfernt. Das zurückbleibende Öl wird mit Essigester behandelt, der unlösliche Rückstand abgetrennt und das Lösungsmittel entfernt. Das zurückbleibende Öl wird in 25 ml Ethanol aufgenommen und mit 1,15 g Hydrazinhydrat (0,023 mol) versetzt. Die Lösung wird 30 Minuten bei 80 °C gerührt. Nach Zugabe von 3 ml konzentrierter Salzsäure wird weitere 30 Minuten bei 80 °C gerührt. Anschließend wird mit 10 ml Wasser versetzt und filtriert. Nach dem Einengen und Umkristallisieren aus Ethanol erhält man das gewünschte Produkt als farblose Kristalle.

Fp: 155-156 °C; Ausbeute: 1,8 g, 27 % der Theorie

Weitere Verbindungen der Formel II, die nach dem gleichen Verfahren in Form ihrer Hydrochloride erhalten wurden, sind in der nachfolgenden Tabelle A aufgeführt.

Tabelle A

| Nr. | $R^1$ | $R^2$ | phys. Daten [Fp. (°C) oder $^1H$-NMR] |
|---|---|---|---|
| II-1 | $CH_3$ | H | 155-156 |
| II-2 | $CH_2CH_3$ | H | 60 |
| II-3 | $CH_2C_6H_5$ | H | 139-140 |
| II-4 | $-CH_2-CH=CH_2$ | H | 94- 95 |
| II-5 | 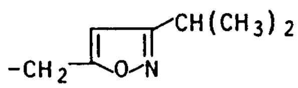 | H | 138 |
| II-6 | 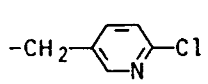 | H | $^1H$-NMR (DMSO) δ: 8,94(s,1H), 8,19(bs,3H), 8,01(d,1H), 7,60(d,1H), 5,30(s,2H), 3,37 (t,2H), 2,94(m,2H), 2,04(m,2H) |
| II-7 | 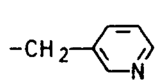 | H | $^1H$-NMR (DMSO) δ: 9,06(s,1H), 8,94(d,1H), 8,63(d,1H), 8,25(bs,3H), 8,06(t,1H), 5,40 (s,2H), 3,31(t,2H), 2,92(m,2H), 2,03(m,2H) |
| II-8 | $-CH_2-[4-Cl-C_6H_4]$ | H | 184-186 |
| II-9 | 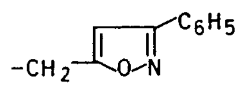 | H | 165-166 |
| II-10 | $-CH_2-(CH_2)_2-O-C_6H_5$ | H | $^1H$-NMR (DMSO) δ: 8,30(bs,3H), 7,33(m,2H), 6,97(m,3H), 4,36(t,2H), 4,06(t,2H), 3,32 (t,2H), 2,96(m,2H), 2,09(m,4H) |
| II-11 | $-CH_2-[3-Cl-C_6H_4]$ | H | 169-170 |

B: Herstellung der Wirkstoffe

Beispiel 1

Herstellung von Ethyl-2-(hexahydro-1-methoxy-pyrimidin-2-yliden)-2-nitroacetat

Zu einer Lösung von 44 g (0,58 mol) Schwefelkohlenstoff in 90 ml Ethanol werden langsam 30 g (0,29 mol) 1-Amino-3-methoxyaminopropan gegeben und 1 Stunde bei Raumtemperatur gerührt. Anschließend werden 7 ml konzentrierte Salzsäure zugegeben und die Lösung 5 Stunden am Rückfluß erhitzt. Das nach dem Abkühlen ausgefallene Hexahydro-1-methoxy-2-pyrimidinthion (VIIa-1) wird abgetrennt und im Vakuum getrocknet.
Fp.: 124 °C; Ausbeute: 22,5 g, 54 % der Theorie
Zu einer Lösung von 13,6 g (0,09 mol) VIIa-1 in Tetrahydrofuran werden zunächst 12 g Natriumcarbonat und anschließend langsam 16 g (0,11 mol) Methyliodid gegeben und die Mischung 2 Stunden am Rückfluß erhitzt. Zur Aufarbeitung wird von den Salzen abfiltriert und das Lösungsmittel entfernt. Der Rückstand wird in Ethylacetat aufgenommen und mit Wasser extrahiert. Die organische Phase wird mit Natriumsulfat getrocknet und das Lösungsmittel entfernt. Man erhält das 1-Methoxy-2-methylthio-1,4,5,6-tetrahydro-pyrimidin (VII-1) als schwach gelbliches Öl.
$^1H$-NMR ($CDCl_3$) δ 3,7 (s,3H), 3,44 (m,4H), 2,27 (s,3H), 1,96 (m, 2H);
Ausbeute: 13,9 g, 93 % der Theorie
2,0 g (0,013 mol) VII-1 werden unter Argon-Schutzgasatmosphäre auf 120 °C erhitzt und langsam 1,8 g

(0,014 mol) Ethylnitroacetat zugegeben. Die Mischung wird noch 1 Stunde bei 120 °C gerührt. Nach dem Abkühlen wird mit einem Gemisch aus Ethanol und Methylenchlorid (4:1) verdünnt und mit Aktivkohle versetzt. Anschließend wird filtriert, das Lösungsmittel entfernt und der verbleibende Rückstand aus Isopropanol umkristallisiert. Man erhält die Titelverbindung als schwach gelbliche Kristalle.
Fp.: 177-178 °C; Ausbeute: 0,7 g, 23 % der Theorie

Beispiel 2

Herstellung von Hexahydro-1-methoxy-2-nitromethylen-pyrimidin

2,0 g (0,011mol) 1-Amino-3-methoxyaminopropan Dihydrochlorid und 2,53 (0,023mol) Kalium-tert.-butylat werden in 120 ml Ethanol 1 h am Rückfluß erhitzt. Anschließend werden 1,86 g (0,011mol) 1,1-Bis-(methylthio)-2-nitroethylen zugesetzt und weitere 6 Stunden am Rückfluß erhitzt bis keine Gasentwicklung mehr beobachtet wird. Zur Aufarbeitung wird von den Salzen abfiltriert und das Lösungsmittel entfernt. Das Rohprodukt wird durch eine Flash-Chromatographie mit Toluol/Ethanol als Fließmittel gereinigt. Man erhält das gewünschte Produkt als schwach gelbliche Kristalle. Fp.: 84-85 °C; Ausbeute: 1,5 g, 77% der Theorie.

Tabelle 1

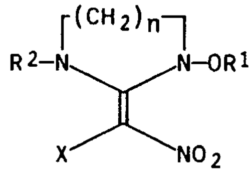

I

| Nr. | $R^1$ | $R^2$ | X | n | phys. Daten [Fp. (°C) oder $^1$H-NMR] |
|---|---|---|---|---|---|
| 1 | $-CH_3$ | H | $-COOC_2H_5$ | 1 | 177-178 |
| 2 | $-CH_3$ | H | H | 1 | 84- 85 |
| 3 | $-CH_2CH_3$ | H | H | 1 | 126-127 |
| 4 | $-CH_3$ | H | H | 0 | >200 |
| 5 | $-CH_2C_6H_5$ | H | H | 1 | 114 |
| 6 | $-CH_2-CH=CH_2$ | H | H | 1 | 110 |
| 7 | 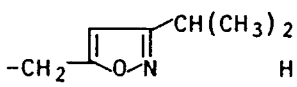 | H | H | 1 | 98 |
| 8 | $-CH_2-[3-Cl-C_6H_4]$ | H | H | 1 | 86- 87 |
| 9 | $-CH_2-[4-Cl-C_6H_4]$ | H | H | 1 | 165-166 |
| 10 | 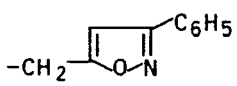 | H | H | 1 | 119-120 |
| 11 | 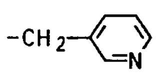 | H | H | 1 | 101 |
| 12 | 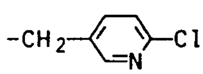 | H | H | 1 | 158 |
| 13 | $-CH_2-(CH_2)_2-O-C_6H_5$ | H | H | 1 | $^1$H-NMR ($CDCl_3$) δ: 10,19 (bs,1H), 7,29 (m,2H), 6,99-6,86 (m,4H), 4,09 (m,2H), 3,59 (m,2H), 3,38 (m,2H), 2,12 (m,4H) |

Tabelle 1 (Fortsetzung)

| Nr. | $R^1$ | $R^2$ | X | n | phys. Daten [Fp. (°C) oder $^1$H-NMR] |
|---|---|---|---|---|---|
| 14 | $-CH_2$ | H | $-CH_2-S-[4-CH_3-C_6H_4]$ | 1 | 96 |
| 15 | $-CH_2-[3-F-C_6H_4]$ | H | H | 1 | 88 |
| 16 | $-CH_2-[2-F-C_6H_4]$ | H | H | 1 | 155 |
| 17 | $-CH_2-[2,6-F-C_6H_3]$ | H | H | 1 | 158 |
| 18 | $-CH_2-[3-CF_3-C_6H_4]$ | H | H | 1 | 128 |
| 19 | $-CH_2-[2-Cl-C_6H_4]$ | H | H | 1 | 193 |

Anwendungsbeispiele

Die insektizide Wirkung der Verbindungen der allgemeinen Formel I ließ sich durch folgende Versuche zeigen:

Die Wirkstoffe wurden

a) als 0,1 %-ige Lösung in Aceton oder

b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

Bei der Untersuchung der Kontaktwirkung gegen a) Prodenia litura (Ägypt. Baumwollwurm) und b) Plutella maculipennis (Kohlschaben-Raupe) zeigten die Verbindungen 2 und 3 Wirkschwellen von a) 0,1 bzw. 0,04 mg und b) von 400 ppm.

## Patentansprüche

**1.** 1-Alkoxy-1,3-diazacycloalkanderivate der allgemeinen Formel I

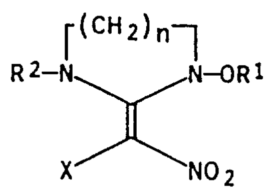

I

in der der Index und die Substitutenten folgende Bedeutung haben:

n

0 oder 1;

$R^1$

$C_1$-$C_{10}$-Alkyl, $C_1$-$C_6$-Alkylcarbonyl, $C_3$-$C_6$-Cycloalkylcarbonyl, $C_3$-$C_6$-Alkenyl oder $C_3$-$C_6$-Alkinyl, wobei diese Gruppen ein bis fünf Halogenatome und/oder einen oder zwei der folgenden Reste tragen können:

- $C_3$-$C_6$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_6$-Alkylamino, $C_1$-$C_6$-Dialkylamino, $C_1$-$C_4$-Alkylcarbonyl, $C_3$-$C_6$-Cycloalkylcarbonyl, $C_1$-$C_4$-Alkylcarbonyloxy oder $C_3$-$C_6$-Cycloalkylcarbonyloxy,
- Phenylcarbonyl, Phenylcarbonyloxy, Phenyl, Phenoxy oder Phenylthio, wobei die Phenylreste ihrerseits eine bis fünf Halogenatome und/oder ein bis drei der folgenden Gruppen tragen können: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;
- einen 5- bis 6-gliedrigen heterocyclischen, aliphatischen oder aromatischen Rest, enthaltend ein bis drei Heteroatome, ausgewählt aus der Gruppe Sauerstoff, Schwefel und Stickstoff, wobei dieser Rest einen oder zwei der folgenden Substituenten tragen kann: Halogen, $C_1$-$C_4$-Alkyl, $C_3$-$C_6$-Cycloalkyl, $C_1$-$C_4$-Alkoxy und Phenyl, wobei die Phenylreste ihrerseits ein bis fünf Halogenatome und/oder ein bis drei der folgenden Gruppen tragen können: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

oder eine Gruppe -C(=O)-R, wobei

R

für Phenyl oder einen 5- bis 6-gliedrigen heterocyclischen, aliphatischen oder aromatischen Rest steht, der ein bis drei Heteroatome, ausgewählt aus der Gruppe Sauerstoff, Schwefel und Stickstoff enthält, wobei diese Reste einen oder zwei der folgenden Substituenten tragen können: Cyano, Nitro, Halogen, $C_1$-$C_4$-Alkyl, $C_1$-$C_4$-Alkoxy, $C_1$-$C_4$-Halogenalkoxy, $C_1$-$C_4$-Alkylthio, $C_1$-$C_4$-Halogenalkylthio und Phenyl;

X

Wasserstoff; Halogen; $C_1$-$C_4$-Alkoxy; $C_1$-$C_4$-Alkylthio; $C_1$-$C_6$-Alkylcarbonyl; $C_1$-$C_6$-Halogenalkylcarbonyl; $C_1$-$C_6$-Alkoxycarbonyl;

$C_1$-$C_4$-Alkyl, welches ein bis neun Halogenatome und/oder ein bis drei der folgenden Gruppen tragen kann: Hydroxy, $C_1$-$C_4$-Alkoxy, $C_1$-$C_4$-Alkylthio, Phenoxy und Phenylthio;

Phenyl, Phenoxy, Phenylthio, Benzoyl oder Phenyloxycarbonyl, wobei die Phenylreste ihrerseits ein bis fünf Halogenatome und/oder ein bis drei der folgenden Gruppen tragen können: Cyano, Nitro, $C_1$-$C_4$-Alkyl und $C_1$-$C_4$-Alkoxy;

$R^2$

Wasserstoff oder $C_1$-$C_4$-Alkyl.

2. 1-Alkoxy-1,3-diazaycloalkanderivate der allgemeinen Formel I gemäß Anspruch 1, in der der Index n 1 bedeutet und die Reste X und $R^2$ für Wasserstoff stehen.

3. Verfahren zur Herstellung der Verbindungen I gemäß Anspruch 1 dadurch gekennzeichnet, daß man ein 1-Alkoxyamino-2- oder 3-aminoalkan der allgemeinen Formel II

$R^2$-NH-$(CH_2)_{n+2}$-NH-$OR^1$ II

in an sich bekannter Weise in einem organischen Lösungsmittel mit einem 1,1-Bisalkylthioalken der allgemeinen Formel III,

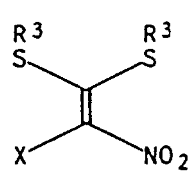

III

in der $R^3$ für eine Alkyl- oder Benzylgruppe steht, umsetzt.

4. Verfahren zur Herstellung der Verbindungen I gemäß Anspruch 1, bei denen $R^2$ für Wasserstoff und X für Alkylcarbonyl, Alkoxycarbonyl, Benzoyl oder Phenyloxycarbonyl steht, dadurch gekennzeichnet, daß man ein 1-Alkoxy-1,3-diaza-2-methylthio-cycloalkanderivat der allgemeinen Formel VII

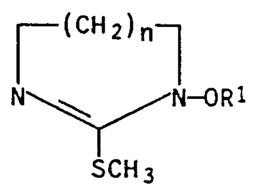

VII

mit einer Nitroverbindung der allgemeinen Formel VIII

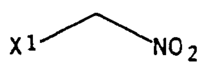

VIII

in der $X^1$ für Alkylcarbonyl, Alkoxycarbonyl, Benzoyl oder Phenyloxycarbonyl steht, umsetzt.

**5.** 1-Alkoxyamino-3-aminopropane der allgemeinen Formel IIa

$R^2$-NH-$(CH_2)_3$-NH-$OR^1$  IIa

und deren Säureadditionssalze, in denen $R^1$ und $R^2$ die in Anspruch 1 angegebenen Bedeutungen haben, wobei $R^1$ nicht Benzyl oder substituiertes Benzyl bedeutet.

**6.** Verfahren zur Herstellung der Verbindungen IIa gemäß Anspruch 5, dadurch gekennzeichnet, daß man ein Alkylamin der allgemeinen Formel IIb

Y-$(CH_2)_3$-N($R^2$)-$T^1$  IIb

in der Y für eine nucleofuge Abgangsgruppe und $T^1$ für eine Amino-Schutzgruppe steht, zunächst in an sich bekannter Weise in einem inerten organischen Lösungsmittel mit einem Hydroxylamin der allgemeinen Formel IV

$T^2$-NH-$OR^1$  IV

in der $T^2$ für Wasserstoff oder eine Aminoschutzgruppe steht, umsetzt und anschließend die Schutzgruppe in an sich bekannter Weise abspaltet.

**7.** Verfahren zur Herstellung der Verbindungen IIa gemäß Anspruch 5, dadurch gekennzeichnet, daß man ein 1-Amino-3-hydroxyaminopropan der allgemeinen Formel IIc

$T^1$-N($R^2$)-$(CH_2)_3$-N(OH)-$T^1$  IIc

in der $T^1$ die in Anspruch 6 gegebene Bedeutung hat, in an sich bekannter Weise in einem inerten organischen Lösungsmittel mit einem Alkylierungsmittel der allgemeinen Formel VI

$R^1$-Y  VI

in der Y für eine nucleofuge Abgangsgruppe steht, umsetzt.

**8.** Schädlingsbekämpfungsmittel, enthaltend eine wirksame Menge eines 1-Alkoxy-1,3-diazacycloalkans der allgemeinen Formel I gemäß Anspruch 1.

**9.** Verfahren zur Bekämpfung von Schädlingen, dadurch gekennzeichnet, daß man die Schädlinge und/oder deren Lebensraum mit einer wirksamen Menge eines 1-Alkoxyl-1,3-diazacycloalkanderivates der allgemeinen Formel I gemäß Anspruch 1 behandelt.

**10.** Verwendung von 1-Alkoxy-1,3-diazacycloalkanderivaten der allgemeinen Formel I gemäß Anspruch 1 zur Bekämpfung von Schädlingen.

## Claims

**1.** A 1-alkoxy-1,3-diazacycloalkane derivative of the formula I

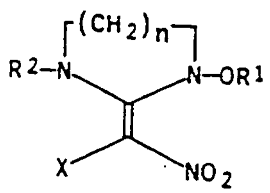

I

where
n is 0 or 1;
$R^1$ is $C_1$-$C_{10}$-alkyl, $C_1$-$C_6$-alkylcarbonyl, $C_3$-$C_6$-cycloalkylcarbonyl, $C_3$-$C_6$-alkenyl or $C_3$-$C_6$-alkynyl, where these groups may carry from one to five halogen atoms or one or two of the following radicals:

- $C_3$-$C_6$-cycloalkyl, $C_1$-$C_4$-alkoxy, $C_1$-$C_4$-haloalkoxy, $C_1$-$C_4$-alkylthio, $C_1$-$C_4$-haloalkylthio, $C_1$-$C_6$-alkylamino, $C_1$-$C_6$-dialkylamino, $C_1$-$C_4$-alkylcarbonyl, $C_3$-$C_6$-cycloalkylcarbonyl, $C_1$-$C_4$-alkylcarbonyloxy or $C_3$-$C_6$-cycloalkylcarbonyloxy,
- phenylcarbonyl, phenylcarbonyloxy, phenyl, phenoxy or phenylthio, where the phenyl radicals in turn may carry from one to five halogen atoms and/or from one to three of the following groups: cyano, nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio;
- a 5-membered or 6-membered heterocyclic, aliphatic or aromatic radical containing from one to three hetero atoms selected from the group consisting of oxygen, sulfur and nitrogen, where this radical may carry one or two of the following substituents: halogen, $C_1$-$C_4$-alkyl, $C_3$-$C_6$-cycloalkyl, $C_1$-$C_4$-alkoxy and phenyl, where the phenyl radicals in turn may carry from one to five halogen atoms and/or from one to three of the following groups: cyano, nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-haloalkylthio;

or -C(=O)-R, where
R is phenyl or a 5-membered or 6-membered heterocyclic, aliphatic or aromatic radical which contains from one to three hetero atoms selected from the group consisting of oxygen, sulfur and nitrogen, where these radicals may carry one or two of the following substituents: cyano, nitro, halogen, $C_1$-$C_4$-alkyl, $C_1$-$C_4$-alkoxy, $C_1$-$C_4$-haloalkoxy, $C_1$-$C_4$-alkylthio, $C_1$-$C_4$-haloalkylthio and phenyl;
X is hydrogen; halogen, $C_1$-$C_4$-alkoxy; $C_1$-$C_4$-alkylthio; $C_1$-$C_6$-alkylcarbonyl; $C_1$-$C_6$-haloalkylcarbonyl; $C_1$-$C_6$-alkoxycarbonyl;
$C_1$-$C_4$-alkyl which may carry from one to nine halogen atoms and/or from one to three of the following groups: hydroxyl, $C_1$-$C_4$-alkoxy, $C_1$-$C_4$-alkylthio, phenoxy and phenylthio;
phenyl, phenoxy, phenylthio, benzoyl or phenyloxycarbonyl, where the phenyl radicals in turn may carry from one to five halogen atoms and/or from one to three of the following groups: cyano, nitro, $C_1$-$C_4$-alkyl and $C_1$-$C_4$-alkoxy; and
$R^2$ is hydrogen or $C_1$-$C_4$-alkyl.

**2.** A 1-alkoxy-1,3-diazacycloalkane derivative of the formula I as claimed in claim 1, where n is 1 and X and $R^2$ are each hydrogen.

**3.** A process for the preparation of a compound I as claimed in claim 1, wherein a 1-alkoxyamino-2- or -3-aminoalkane of the formula II

$R^2$-NH-$(CH_2)_{n+2}$-NH-$OR^1$ II

is reacted in a conventional manner with a 1,1-bisalkylthioalkene of the formula III

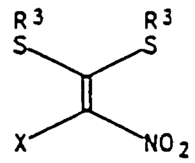

III

where $R^3$ is alkyl or benzyl, in an organic solvent.

4. A process for the preparation of a compound I as claimed in claim 1, in which $R^2$ is hydrogen and X is alkylcarbonyl, alkoxycarbonyl, benzoyl or phenyloxycarbonyl, wherein a 1-alkoxy-1,3-diaza-2-methylthiocycloalkane derivative of the formula VII

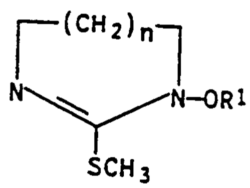

VII

is reacted with a nitro compound of the formula VIII

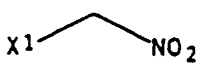

VIII

where $X^1$ is alkylcarbonyl, alkoxycarbonyl, benzoyl or phenyloxycarbonyl.

5. A 1-alkoxyamino-3-aminopropane of the formula IIa

$R^2$-NH-$(CH_2)_3$-NH-$OR^1$ IIa

and acid addition salts thereof in which $R^1$ and $R^2$ have the meanings stated in claim 1 but $R^1$ is not benzyl or substituted benzyl.

6. A process for the preparation of a compound IIa as claimed in claim 5, wherein an alkylamine of the formula IIb

Y-$(CH_2)_3$-N($R^2$)-$T^1$ IIb

where Y is a nucleofugic leaving group and $T^1$ is a protective group for amino, is first reacted in a conventional manner with a hydroxylamine of the formula IV

$T^2$-NH-$OR^1$ IV

where $T^2$ is hydrogen or a protective group for amino, in an inert organic solvent, and the protective group is then eliminated in a conventional manner.

7. A process for the preparation of a compound IIa as claimed in claim 5, wherein a 1-amino-3-hydroxylaminopropane of the formula IIc

$T^1$-N($R^2$)-$(CH_2)_3$-N(OH)-$T^1$ IIc

where $T^1$ has the meanings stated in claim 6, is reacted in a conventional manner with an alkylating

agent of the formula VI

$R^1$-Y VI

where Y is a nucleofugic leaving group, in an inert organic solvent.

**8.** A pesticide containing an effective amount of a 1-alkoxy-1,3-diazacycloalkane of the formula I as claimed in claim 1.

**9.** A method for controlling pests, wherein the pests and/or their habitat are or is treated with an effective amount of a 1-alkoxy-1,3-diazacycloalkane derivative of the formula I as claimed in claim 1.

**10.** Use of a 1-alkoxy-1,3-diazacycloalkane derivative of the formula I as claimed in claim 1 for controlling pests.

## Revendications

**1.** Dérivés de 1-alcoxy-1,3-diazocycloalcanes de formule générale I

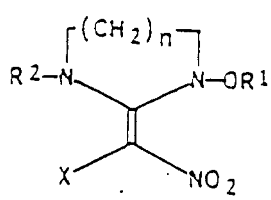

I

dans laquelle l'indice et les symboles ont les significations suivantes :
n est égal à 0 ou 1 ;
$R^1$ représente
un groupe alkyle en C1-C10, (alkyle en C1-C6)carbonyle, (cycloalkyle en C3-C6)carbonyle, alcényle en C3-C6 ou alcynyle en C3-C6, ces groupes pouvant porter un à cinq atomes d'halogènes et/ou un ou deux groupes suivants :

- cycloalkyle en C3-C6, alcoxy en C1-C4, halogénoalcoxy en C1-C4, alkylthio en C1-C4, halogénoalkylthio en C1-C4, alkylamino en C1-C6, di-(alkyle en C1-C6)amino, (alkyle en C1-C4)-carbonyle, (cycloalkyle en C3-C6)carbonyle, (alkyle en C1-C4)carbonyloxy, (cycloalkyle en C3-C6)carbonyloxy,
- phénylcarbonyle, phénylcarbonyloxy, phényle, phénoxy ou phénylthio, dont les groupes phényle peuvent eux-mêmes porter un à cinq atomes d'halogènes et/ou un à trois des groupes suivants : cyano, nitro, alkyle en C1-C4, halogénoalkyle en C1-C4, alcoxy en C1-C4, halogénoalcoxy en C1-C4, alkylthio en C1-C4 ou halogénoalkylthio en C1-C4;
- un groupe hétérocyclique aliphatique ou aromatique de cinq à six chaînons contenant un à trois hétéroatomes choisis parmi l'oxygène, le soufre et l'azote, ce groupe pouvant porter un ou deux des substituants suivants : les halogènes, les groupes alkyle en C1-C4, cycloalkyle en C3-C6, alcoxy en C1-C4 et phényle, les groupes phényle pouvant eux-mêmes porter un à cinq atomes d'halogènes et/ou un à trois des groupes suivants : cyano, nitro, alkyle en C1-C4, halogénoalkyle en C1-C4, alcoxy en C1-C4, halogénoalcoxy en C1-C4, alkylthio en C1-C4 et halogénoalkylthio en C1-C4 ;

ou un groupe -C(=O)-R dans lequel
R représente
un groupe phényle ou un groupe hétérocyclique aliphatique ou aromatique de cinq à six chaînons contenant un à trois hétéroatomes choisis parmi l'oxygène, le soufre et l'azote, ces groupes pouvant porter un ou deux des substituants suivants : cyano, nitro, halogéno, alkyle en C1-C4, alcoxy en C1-C4, halogénoalcoxy en C1-C4, alkylthio en C1-C4, halogénoalkylthio en C1-C4 et phényle ;
X représente
l'hydrogène ; un halogène ; un groupe alcoxy en C1-C4 ; alkylthio en C1-C4 ; (alkyle en C1-C6)-carbonyle ; (halogénoalkyle en C1-C6)carbonyle ; (alcoxy en C1-C6)carbonyle ;
un groupe alkyle en C1-C4 qui peut porter un à neuf atomes d'halogènes et/ou un à trois des

groupes suivants : hydroxy, alcoxy en C1-C4, alkylthio en C1-C4, phénoxy et phénylthio ;

un groupe phényle, phénoxy, phénylthio, benzoyle ou phényloxycarbonyle dont les noyaux phényle peuvent eux-mêmes porter un à cinq atomes d'halogènes et/ou un à trois des groupes suivants : cyano, nitro, alkyle en C1-C4 et alcoxy en C1-C4 ;

$R^2$ représente

l'hydrogène ou un groupe alkyle en C1-C4.

2. Dérivés de 1-alcoxy-1,3-diazacycloalcanes de formule générale I selon la revendication 1, dans laquelle n est égal à 1 et les symboles X et $R^2$ représentent l'hydrogène.

3. Procédé de préparation des composés I de la revendication 1, caractérisé en ce que l'on fait réagir un 1-alcoxyamino-2- ou -3-aminoalcane de formule générale II

$R^2\text{-NH-}(CH_2)_{n+2}\text{-NH-OR}^1$ II

de manière connue en soi, dans un solvant organique, avec un 1,1-bisalkylthioalcène de formule générale III

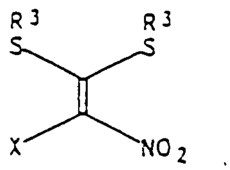

III

dans laquelle $R^3$ représente un groupe alkyle ou benzyle.

4. Procédé de préparation des composés I de la revendication 1, pour lesquels $R^2$ représente l'hydrogène et X un groupe alkylcarbonyle, alcoxycarbonyle, benzoyle ou phényloxycarbonyle, caractérisé en ce que l'on fait réagir un dérivé de 1-alcoxy-1,3-diaza-2-méthylthiocycloalcane de formule générale VII

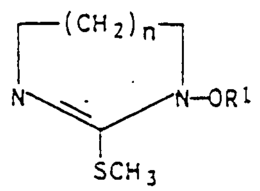

VII

avec un dérivé nitré de formule générale VIII

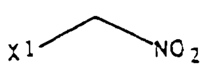

VIII

dans laquelle $X^1$ représente un groupe alkylcarbonyle, alcoxycarbonyle, benzoyle ou phényloxycarbonyle.

5. 1-alcoxyamino-3-aminopropanes de formule générale IIa

$R^2\text{-NH-}(CH_2)_3\text{-NH-OR}^1$ IIa

et leurs sels formés par addition avec des acides, pour lesquels $R^1$ et $R^2$ ont les significations indiquées dans la revendication 1, sous réserve que $R^1$ ne peut représenter un groupe benzyle ou un groupe benzyle substitué.

6. Procédé de préparation des composés IIa de la revendication 5, caractérisé en ce que l'on fait réagir une alkylamino de formule générale IIb

$Y-(CH_2)_3-N(R^2)-T^1$ IIb

dans laquelle Y représente un groupe éliminable nucléofuge et $T^1$ représente un groupe protecteur du groupe amino, d'abord, de manière connue en soi, dans un solvant organique inerte, avec une hydroxylamine de formule générale IV

$T^2-NH-OR^1$ IV

dans laquelle $T^2$ représente l'hydrogène ou un groupe protecteur du groupe amino, après quoi on scinde le groupe protecteur de manière connue en soi.

7. Procédé de préparation des composés IIa de la revendication 5, caractérisé en ce que l'on fait réagir un 1-amino-3-hydroxyaminopropane de formule générale IIc

$T^1-N(R^2)-(CH_2)_3-N(OH)-T^1$ IIc

dans laquelle $T^1$ a les significations indiquées dans la revendication 6, de manière connue en soi, dans un solvant organique inerte, avec un agent alkylant de formule générale VI

R1-Y VI

dans laquelle Y représente un groupe éliminable nucléofuge.

8. Produit parasiticide contenant une quantité efficace d'un 1-alcoxy-1,3-diazacycloalcane de formule générale I de la revendication 1.

9. Procédé pour combattre les parasites, caractérisé en ce que l'on traite les parasites et/ou leur habitat par une quantité efficace d'un dérivé de 1-alcoxy-1,3-diazacycloalcane de formule générale I de la revendication 1.

10. Utilisation des dérivés de 1-alcoxyl-1,3-diazacycloalcanes de formule générale I de la revendication 1 pour la lutte contre les parasites.